# EUROPÄISCHE PATENTSCHRIFT

(11) **EP 4 094 926 B1**
(45) Veröffentlichungstag und Bekanntmachung des Hinweises auf die Patenterteilung: **31.01.2024**
(21) Anmeldenummer: 21176035.0
(22) Anmeldetag: 26.05.2021
(51) Int. Cl.: B29C 64/135, B29C 64/393, B29C 64/165, B33Y 10/00, B33Y 30/00, B33Y 50/02, B33Y 70/00, B33Y 80/00

(54) **VORRICHTUNG, SYSTEM UND VERWENDUNG DERSELBEN SOWIE VERFAHREN ZUM ERZEUGEN EINER 3D-STRUKTUR**
DEVICE, SYSTEM AND USE OF SAME AND METHOD FOR PRODUCING A 3D STRUCTURE
DISPOSITIF, SYSTÈME ET SON UTILISATION, AINSI QUE PROCÉDÉ DE GÉNÉRATION D'UNE STRUCTURE 3D

(43) Veröffentlichungstag der Anmeldung: 30.11.2022
(73) Patentinhaber: uppolluX GmbH & Co. KG, 78532 Tuttlingen (DE)
(72) Erfinder: Baumann, Daniel, 78532 Tuttlingen (DE)
(74) Vertreter: Kohler Schmid Möbus Patentanwälte

(56) Entgegenhaltungen:
- EP-A1- 3 292 990
- WO-A2-2021/055063
- CN-A- 109 605 742
- US-A1- 2018 273 776

## Beschreibung

Die vorliegende Erfindung betrifft eine Vorrichtung, ein System und ein Verfahren zum Erzeugen einer dreidimensionalen (=3D) Struktur. Darüber hinaus betrifft die Erfindung eine Verwendung der Vorrichtung sowie des Systems.

Beim sogenannten 3D-Druck (=additive Manufacturing) erfolgt die Fertigung einer dreidimensionalen Struktur durch einen schichtweisen Aufbau computergesteuert aus einem oder mehreren flüssigen oder festen Werkstoffen nach vorgegebenen Maßen und Formen. Beim Aufbau finden physikalische oder chemische Härtungs- und/oder Schmelzprozesse statt. Typische Werkstoffe für das 3D-Drucken sind Kunststoffe, Kunstharze, Keramiken und Metalle. Der 3D-Druck zeichnet sich durch eine kostengünstige Individualisierbarkeit der 3D-Struktur hinsichtlich Geometrie, Materialität und Funktionalität bei zugleich fertigungstechnisch hoher Automatisier- und Dezentralisierbarkeit aus. Auf der Basis CAD-optimierter Bilddateien gelingt somit heute mittels unterschiedlichster additiver Verfahrenstechnologien eine kontrollierte, reproduzierbare und in Echtzeit modulierbare Herstellung dreidimensionaler Strukturen durch eine zunehmend präzise Materialdeosition in einem baulanadatierten Koordinatensystem.

Entsprechender Stand der Technik findet sich in US 2018/273776 A1, CN 109 605 742 A, EP 3 292 990 A1 und WO 2021/055063 A2.

Im Bereich der Medizin stellt der Ersatz funktioneller Zellkomplexe und mechanisch-stützender Gerüststrukturen eine große Herausforderung dar. Gewebeverluste aufgrund von Erkrankungen, Verletzungen, chronischem Verschleiß, sowie (radikalen) chirurgischen Eingriffen können nicht immer durch körpereigene Selbstheilungskräfte repariert werden. Die hierdurch eingeschränkte Funktionalität und Stabilität der betroffenen Gewebe, ihrer Nachbarstrukturen und ggf. des gesamten Körpers führen häufig zu einer erheblichen Minderung der Lebensqualität der Betroffenen sowie zu funktionellen Einschränkungen.

Bis zum heutigen Tag gilt in allen rekonstruktiven Disziplinen der Medizin der humane, autologe Gewebeersatz als Optimum. Dieser bietet die charakteristischen Vorteile des Originalgewebes und eine maximale Gewebekompatiblität aufgrund identischer immunologischer Oberflächenmerkmale. Diese Ressource ist jedoch begrenzt.

Intrakorporale Stimulationsverfahren wie die Mikrofrakturierung führen häufig zu mechanisch instabilem Reparationsgewebe, z.B. Faserknorpel, und letztlich zu einer Defektheilung und Sekundärarthrose.

Verfahren zur extrakorporalen Gewebevermehrung in Bioreaktoren sind aufwändig, teuer und hinsichtlich der Authentizität ihrer biochemischen und biomechanischen Stimuli insbesondere für die simultane Aufzucht verschiedener Zelllinien bislang als insuffizient zu bewerten. Dies gilt insbesondere für die Bebrütung komplexer 3D-Bioprint-Produkte, deren Zellverbunde zwischenzeitlich erheblich an Detailauflösung gewonnen haben, jedoch u.a. unter einer fehlenden physiologischen Frühadaptation leiden.

Betrachtet man die aktuell zum Einsatz kommenden Bioprint-Technologien, so beeinträchtigen Scherkräfte und Druckgefälle der Extrusionsverfahren und lange Fertigungszeiten der Extrusions- und Mikroventilverfahren die Zellvitalität, rheologische Einschränkungen die Ortsauflösung und Zelldichte.

SLA- und LASER-Verfahren wiederum weisen bei hoher Detailauflösung und Zelldichte zytotoxische, mutagene und zellmetabolische Nebenwirkungen auf. Alle gängigen Bioprint-Systeme sind darüber hinaus in vielerlei Hinsicht für den direkten klinischen Einsatz limitiert. Dies kann durch eine begrenzte Eindringtiefe und mangelnde geometrische Adaptivität aufgrund starrer Achsenmechaniken oder ungerichteter Welleninterferenzen, insbesondere aber auch aufgrund ihrer noch begrenzten Zahl simultan prozessierbarer Zell- und Materialtypen, bedingt sein. Letztlich resultiert dies in einer insuffizienten Stabilität und Funktionalität ihrer Produkte.

Künstliche Endoprothesen wiederum stellen seit jeher einen Kompromiss aus mechanischer Haltbarkeit und artifizieller Biokompatibilität dar. Deren individuelle Konfektionierung erfolgt in der medizinischen Regelversorgung bislang nur durch Einsatz von Standard-Größen und Standard-Materialien, fernab jeglicher Bioauthentizität. Wenngleich kommerziell überaus populär und stabil, weisen diese Implantate keinerlei Anpassungs- oder Wachstumsvermögen auf und erfordern häufig invasive Wechseloperationen.

Alle genannten Strategien zum Gewebeersatz teilen unabhängig von ihren spezifischen Nachteilen den kardinalen Grundkonflikt der konkurrierenden Interessen von Stabilität und Minimalinvasivität. Dies deshalb, weil mit zunehmender Stabilität des Implantates unweigerlich die Invasivität seiner Implantation und folglich das Risiko perioperativer Komplikationen steigt. Kollateralschäden der Umgebungsgewebe sind dabei entlang des operativen Zugangsweges, bei der Gewebeentnahme und insbesondere im Rahmen der Implantat-Einbringung durch die Notwendigkeit der traumatischen Kongruenz-Anpassung von Implantat und Implantat-Bett und invasive Fixierungstechniken unvermeidbar. Einzeln und in Summe schränken diese Kollateralschäden das lokale Heilungsvermögen ein - insbesondere bei bereits vorbestehend reduzierten Regenerationskapazitäten des Patienten. Dies kann in einer akut oder chronisch inadäquaten Gewebestabilität und -Vitalität, pathologischen Biomechanik und erhöhten Wahrscheinlichkeit von Folgemorbiditäten resultieren.

Als für die Implantat-Einheilung besonders dramatisch zu bewerten ist hierbei, dass die adaptiven Geweberesektionen, aber auch die mechanischen Verankerungsmechanismen (Naht, Schrauben, Pins, press-fit) im Speziellen die Übergangszone (Interface) vom Implantat zum Organismus schädigen, welcher eine Schlüsselfunktion für die Implantat-Integration zukommt. Strukturelle Läsionen der Übergangszone resultieren unmittelbar in einer eingeschränkten biomimetischen Potenz der Implantat-Oberfläche. Auch kann es zu einer vermehrten Freisetzung von Entzündungsmediatoren mit hernach kaskadisch getriggerten, überaus unerwünschten Imbalancen der Mikrozirkulation, Permeabilität, Sauerstoff- und Näherstoff-Diffusion, Zelladhäsion und -migration kommen. Eine sehr geringe Implantat-Stabilität wiederum erleichtert zwar die formkongruente Auskleidung des zu versorgenden Gewebedefektes - und somit die lokale Adhäsion - ist aber zumindest für den muskuloskelettalen Stütz- und Halteapparat überwiegend als inadäquat zu erachten.

Betrachtet man die Forschungsergebnisse der letzten Jahre der TERM (Tissue Engineering & Regenerative Medizin) zeichnen sich trotz aller experimentellen invitro-Erfolge somit für die klinische Anwendung fundamentale Hürden ab, welche mit den gängigen Lösungsansätzen auch zukünftig kaum zu bewältigen sein werden.

In einem Zeitalter global zunehmend virulenter und multiresistenter nosokomialer Keime rückt zudem die Invasivität und Extensivität chirurgischer Eingriffe als bedeutender Risikofaktor und somit Prädiktor des Patienten-Outcomes vermehrt in den Vordergrund. Während die Kompatibilität bioartifizieller Gewebeimplantate stetig wächst, erfordern traditionelle allogene Gewebeprodukte, aber auch transgene Xenotransplantate weiterhin immunsuppressive Begleitmedikationen, welche das Abwehrvermögen des Empfängers zusätzlich schwächen. Zugleich öffnet sich die Schere zwischen einer zunehmenden diagnostischen Sensitivität und Spezifität, einhergehend mit einem wachsenden Verständnis prämorphologischer Gewebefrühschäden bzw. Mikroinstabilitäten einerseits und deren praktischer Therapierbarkeit mittels klassischer Operationstechniken andererseits. So ist zu befürchten, dass die minimalinvasive Chirurgie alsbald an die Grenzen der technischen Machbarkeit stoßen wird. Eine weitere Minimierung von Kollateralschäden und Folgekomplikationen kann nur im Rahmen neuartiger interventioneller Gewebeersatz-Verfahren gemäß dem ärztlichen Leitprinzip "primum non nocere" gelingen.

Zahllose entwicklungsbiologische Studien der vergangenen Jahre zeugen von der immensen Bedeutung des Informationsgehalts mikrodimensionaler Gerüststrukturen zur Steuerung der Geweberegeneration und Triggerung von Botenstoffkaskaden. So dient die 3D-Mikroarchitektur als fundamentaler, universeller morphologischer Code der biomimetischen Aktivität und folglich als induktiver Schlüsselfaktor der Zell-Adhäsion, -Migration, -Differenzierung und - Proliferation, mit wesentlichem Einfluss der strukturellen Anisotropie auf die Natürlichkeit der zellulären Performance. Die räumliche Vielfalt von Oberflächenmerkmalen, Porengröße und Materialien eines Implantates beeinflusst demnach unweigerlich die lokoregionäre Spezifität, Aktivität und Dominanz differierender Zellpopulationen und abhängig von deren extrazellulären Matrix konsekutiv die mechanischen Eigenschaften der Regenerate bzw. das Schicksal der in unterschiedlichem Maße integrierten - im Idealfall adoptierten - Implantate. Das bioartifizielle 3D-Gerüst dient somit in vivo den Zellen sowohl als mikroökologische Nische - also als Brutkammer, Platzhalter und Gerüst, als auch als Amme - also instruktive Differenzierungshilfe - für das Remodeling. Insgesamt also als Garant eines suffizienten, physiologischen Heilungsprozesses, gekennzeichnet durch einen adäquaten und zielgerichteten Gewebeaufbau und den zeitgerechten sequentiellen Austausch provisorischer gegen authentische Gewebekomplexe, mit dem Ziel einer gesunden Belastbarkeit und physiologischen Funktionalität.

### Aufgabe der Erfindung

Es ist die Aufgabe der Erfindung, eine Vorrichtung sowie ein System anzugeben, mittels derer eine 3D-Struktur auf einfache und hochpräzise Weise in vitro als auch in vivo, d. h. im menschlichen/tierischen Körper, mit einem großen Auflösungsvermögen erzeugt (gefertigt) werden kann. Darüber hinaus ist es die Aufgabe der Erfindung, ein Verfahren zum Erzeugen eines 3D-Struktur sowie Verwendungen der Vorrichtung und des Systems anzugeben.

### Lösung der erfindungsgemäßen Aufgabe

Die die Vorrichtung betreffende Aufgabe wird durch eine Vorrichtung mit den in Anspruch 1 angegebenen Merkmalen und die das System betreffende Aufgabe wird durch ein System mit den in Anspruch 11 angegebenen Merkmalen gelöst. Die Verwendung der Vorrichtung bzw. des Systems ist in Anspruch 22 angegeben. Das erfindungsgemäße Verfahren (MRiP) ist in Anspruch 23 angegebenen.

### Vorbemerkungen

In der nachstehenden Beschreibung der Erfindung sind die Begriffe Präpolymer und Polymervorstufe synonym verwendet.

### Vorrichtung

Die erfindungsgemäße Vorrichtung dient dem Erzeugen einer dreidimensionalen (3D-)Struktur und umfasst:
- einen Magnetfeldgenerator zum Erzeugen eines statischen Magnetfeldes B₀ in einem Arbeitsbereich der Vorrichtung, in dem eine Polymervorstufe mit zumindest einer paramagnetischen Substanz anordenbar ist;
- Gradientenspulen zum Erzeugen von magnetischen Gradientenfeldern in allen drei Raumrichtungen x, y, z, mittels derer die paramagnetische Substanz in einem definierten Voxel V der Polymervorstufe ortscodierbar ist;
- einen Hochfrequenz- (HF-) Feldgenerator zum, bevorzugt gepulsten, Einstrahlen von HF-Strahlung in den Arbeitsbereich; und
- eine Steuerungseinheit, die dazu eingerichtet ist, den HF-Feldgenerator derart anzusteuern, dass die ortskodierte paramagnetische Substanz im Voxel V mittels einer auf die paramagnetische Substanz abgestimmten Feldfrequenz der HF-Strahlung anregbar ist, um die thermische Polymerisation der Polymervorstufe, bevorzugt alleinig, im Voxel V auszulösen.

Die erfindungsgemäße Vorrichtung erlaubt einen additiven induktiven mehrdimensionalen Aufbau von beliebigen 3D- Strukturen. Dies durch präzise HF-Stimulation der paramagnetischen Substanz in elektromagnetischen Resonanznischen, welche ortsspezifisch, insbesondere auf der Basis hochauflösender Bilddaten, durch gezielte Überlagerung magnetischer Gradientenfelder innerhalb einer mehr oder weniger liquiden thermosensitiven Polymervorstufe erzeugt werden. Die Vorrichtung erlaubt dadurch die Fertigung von 3D-Strukturen jedweder Geometrie, Struktur, Oberflächengestaltung und in Abhängigkeit der eingesetzten Materialien auch mit jeweilig vorgegebenen Materialeigenschaften. Ein- und mehrzeitige Nachbearbeitungsprozeduren des 3D-Erzzeugnisses sind möglich.

Die Vorrichtung kann nach der Erfindung insbesondere eine Einrichtung zur Bilddatenakquisition bzw. Bildakquisition umfassen. Mit anderen Worten eine Einrichtung zum Gewinnen von Bilddaten. Besonders bevorzugt umfasst die Vorrichtung ein MRT-Gerät. MRT-Geräte können funktionell durch eine geeignete Programmierung ihrer Steuerungssoftware zu einer erfindungsgemäßen Vorrichtung erweitert werden. Es versteht sich, dass dies grundsätzlich auch bei bereits existierenden MRT-Geräten möglich ist. Darüber hinaus erlaubt das MRT-Gerät die Akquisition von Bilddaten aus dem Arbeitsbereich, mithin der Polymervorstufe, des (ggf. erst teilerzeugten) 3D-Struktur sowie bedarfsweise auch der jeweiligen Umgebung (hier insbesondere Gewebestrukturen in vivo).

Die Vorrichtung kann nach der Erfindung zusätzlich oder alternativ einen Computertomographen (= CT), einen digitalen Volumentomographen (= DVT), ein Sonographiegerät, einen LASER-Scanner und/oder einen Positronen-Emissions-Tomographen (= PET) umfassen, um geeignete Bilddaten aus den vorgenannten Bereichen zu akquirieren.

Die Steuerungseinheit der Vorrichtung weist vorzugsweise einen Betriebsmodus zum Gewinnen und Auswerten von Bilddaten, insbesondere magnetresonanztomografischen Daten, aus dem Arbeitsbereich auf. Dadurch können Bilddaten aus der Polymervorstufe, dem (ggf. erst teilgefertigten) 3D-Struktur sowie bedarfsweise auch der jeweiligen Umgebung (hier insbesondere Gewebestrukturen in vivo) gewonnen werden. Dadurch erlaubt die Vorrichtung, eine bildgebende Diagnostik mit dem additiven Verfahren zum Erzeugen des 3D-Struktur zu kombinieren bzw. die Logik elektromagnetischer und biomimetischer Induktionsprinzipien zu kombinieren.

Umfasst die Vorrichtung ein MRT, so können MRT-gestützt Thermometriedaten aus dem Arbeitsbereich, d. h. der Polymervorstufe, dem (ggf. erst teilgefertigten) 3D-Struktur sowie bedarfsweise auch der jeweiligen Umgebung (hier insbesondere Gewebestrukturen in vivo) gewonnen werden. Diese Thermometriedaten können beim Erzeugen der 3D-Struktur, etwa beim Festlegen der Dauer bzw. der Intensität des zur Anregung eines definierten Voxels einzustrahlenden HF-Felds berücksichtigt werden.

Bei additiven Fertigungsverfahren stellen thermodynamische Phänomene bekanntlich eine wichtige Artefaktequelle dar, deren Effekte es bestmöglich zu beherrschen gilt. Dies, um sowohl mikrodimensionalen Einbußen der Detailauflösung als auch makrodimensionalen Struktur-Inhomogenitäten und - irregularitäten der 3D-Struktur vorzubeugen.

Thermosensitive Sequenzen im Rahmen sogenannter MRT-assistierter HIFUS-Behandlungen (hochfokussierter Ultraschall) haben ihre klinische Praktikabilität und Verlässlichkeit zwischenzeitlich bewiesen. Insbesondere die Protonenresonanz-Methode zeichnet sich dabei durch eine hohe räumliche, zeitliche und thermometrische Auflösung und Reliabilität aus und bietet sich daher auch zur Überwachung des Fertigungsprozesses an, um unvorteilhafte Wärmeableitungen bzw. -kumulationen im dreidimensionalen Raum frühzeitig zu detektieren. Die Vorrichtung kann nach der Erfindung auf die Durchführung solcher thermosensitiven Sequenzen ausgelegt sein.

Die Vorrichtung weist vorzugsweise eine Softwareapplikation auf, mittels derer anhand der im Rahmen der thermosensitiven Sequenzen gewonnenen Daten absolute Temperaturwerte ermittelbar und vorzugsweise farbig codierbar, benutzerdefinierte topographische und thermische Schwellenwerte ortsspezifisch mit Alarmen koppelbar und die Einhaltung präziser Expositionsdosisgrenzen automatisierbar bzw. semiautonom regulierbar sind.

Während einer Verminderung der Detailauflösung bei der Fertigung der 3D-Struktur auf Unschärfen der Polymerisationsgrenzen zurückzuführen ist, welche infolge thermodynamischer, voxelüberschreitender Wärmeleitungen während der Fertigung auftreten, demarkieren sich makrodimensionale Aberrationen auf der Basis grober Wärmeakkumulationen vornehmlich erst nach dem Ende der Polymerisation, resultierend in Schrumpfung und Verzug infolge der Materialrelaxation im Rahmen der Abkühlung. Diese Phänomene sind erfahrungsgemäß bei induktiver Erhitzung geringer ausgeprägt als bei anderen thermischen Härtungsverfahren und können durch Vorwärmen des Präpolymers und Nachwärmen des Polymers weiter reduziert werden. Bei der in-vivo-Fertigung lassen die physiologische Körpertemperatur und die relativ niedrige Transitionsschwellen der Präpolymere im Vergleich zur industriellen (ex-vivo) Fertigung von 3D-Strukturen ohnehin nur sehr geringe Temperaturgefälle erwarten, welche z.B. über akzessorische Wärmequellen (z.B. Infrarot-Dioden, UV-/Laserdioden, energiereiche Strahler, heiße Luft) bzw. eine fraktionierte Erwärmung, optimiert durch Segmentierungsverfahren und Thermometrieschwellen zu beherrschen sein sollten.

Ein Anlassen - also Vorwärmen - des Präpolymers - ggf. auch des gesamten Situs bzw. - in vivo der anatomischen Region - dient dabei sowohl einer Milderung der Temperaturgradienten und Homogenisierung des Temperaturprofils allgemein, als auch speziell der Reduktion induktiv zu applizierender Energiemengen und somit der Risikoreduktion aberranter Wärmedynamiken auf Mikro- und Makroniveau. Je klarer und enger definiert die Transitionsschwelle ist, desto klarer ist die fertigungstechnische Trennschärfe. Darüber hinaus gilt, je geringer die Wärmeleitfähigkeit des eingesetzten Präpolymers + Polymers, desto geringer die Gefahr einer heterotopen Wärmeakkumulation und somit einer dystopen Polymerisation, desto höher also das thermische und folglich das strukturelle Auflösungsvermögen.

Um makrodimensionale wie auch mikrodimensionale Wärmeakkumulationen zu vermeiden, sollte die Generierung großer Soliditäts- und Volumendifferenzen, konzentrierter Materialmassen, starker Kalibersprünge und starker Temperaturdifferenzen im Polymerprodukt vermieden werden. Andererseits steigt im Präpolymer das Auflösungsvermögen mit der Steilheit des Temperaturgradienten zwischen induktiv erwärmten, isolierten Einzelvoxeln relativ zu ihrer Umgebung, weshalb an relevanten Grenzzonen, funktionellen Reliefs und Randkanten sogar kühlende Maßnahmen zur Detailoptimierung zu erwägen sind. Auch eine gewisse Stimulationsredundanz der Oszillatoren bzw. Trägheit der Polymerisation kann die thermische Artefaktanfälligkeit zugunsten des strukturellen Auflösungsvermögens reduzieren. Beim Multi-Shot-Konzept sind entsprechend mehrere stimulative HF-Impulse notwendig, um die Transitionstemperatur zu erreichen, streng abgestimmt auf die thermische Leitfähigkeit des Präpolymers, resultierend in einem steileren Temperaturgefälle zum jeweiligen Nachbarvoxel und somit einer höheren Trennschärfe.

Mit zunehmender Aushärtung des Polymers verändern sich die strukturellen Gegebenheiten mit erheblichem Einfluss auf die Wärmeableitung. Durch zonal variierende Materialkontinuität und -Solidifikation ergibt sich mit fortschreitender Aushärtung des Polymers ein zunehmend heterogenes thermisches System, in welchem durch Kumulationseffekte ein Nebeneinander von überwärmten und unterkühlten Zonen entsteht, die prospektiv einkalkuliert und proaktiv kompensiert werden müssen, um dystope Polymerisationen bestmöglich zu vermeiden. Dies gelingt unter Würdigung aller beschriebenen Phänomene und unter Berücksichtigung aller genannten Einflussfaktoren einerseits durch Auswahl eines geeigneten thermoresponsiven Prepolymer(-Komposits) mit zweckmäßiger Lage der thermischen Transitionsschwelle und vorteilhafter thermischer Leitfähigkeit (s.o.), insbesondere aber durch gezielte Modulation der HF-Impulsdauer und -Intervalle sowie deren zeitlicher und räumlicher Perzeption im dreidimensionalen Raum durch dynamische Adaptation der resonanzvulnerablen Voxelgröße und -position mittels dynamischer Magnetfeldgradienten.

So führt eine längere kontinuierliche lokale Anregung zu einem steileren Temperaturgefälle als eine repetitiv wiederholte kurze, impulsive Anregung, und die sequentielle Anregung zweier unmittelbar benachbarter Voxel führt summarisch zu einer lokal höheren Wärmeakkumulation als die Stimulation zweier voneinander entfernter Voxel.

Die Polymerisation der Polymervorstufe kann ggf. das Oszillationsvermögen - und die Resonanzspezifität der (Nano-)Oszillatoren - selbstterminierend einschränken. Allgemein gilt: Je dicker und größer die zu erzeugende 3D-Struktur und je solider ihr Material, desto höher die Gefahr der Wärmeakkumulation; je kleiner, schlanker und diskontinuierlicher die zu erzeugende 3D-Struktur, desto schneller erfolgt ein Temperaturausgleich. Umso wichtiger ist es, jene hiervon kalkulierbaren Effekte zum frühestmöglichen Zeitpunkt Beachtung zu schenken, sie in die CAD-Konstruktion der zu fertigenden 3D-Struktur und Optimierung der Sequenzalgorithmik einfließen zu lassen und ggf. in Echtzeit adaptiv zu regulieren.

Hierzu gehört auch die Berücksichtigung spezieller Formen und Strukturunterbrüche als Wärmeleiter bzw. thermische Hindernisse in den CAD-Bauplan, um Wärmeleitungsphänomene zu kanalisieren bzw. einzudämmen, gezielt lokal Spannungen zu erzeugen und andernorts abzubauen.

Unabhängig davon führt eine höhere Konzentration der paramagnetischen Substanz bzw. der (Nano-)Oszillatoren-Konzentration des Präpolymers theoretisch zu einem höheren induktiven räumlichen Auflösungsvermögen.

Auch eine Steigerung des prozeduralen Umgebungsdrucks, einhergehend mit einer zugleich reduzierten Präpolymerfluktuation können das induktive räumliche Auflösungsvermögen begünstigen. Zwar entstehen Makroaberrationen schichtübergreifend besonders während der Abkühlung, doch auch innerhalb der Schichten bereits bei der Material-Konsolidierung. Auch diesem Phänomen kann durch Erhöhung des Umgebungsdrucks und die Bereitstellung ausreichender Polymervorstufen-Reserven wirksam entgegengewirkt werden. Mittels der erfindungsgemäßen Vorrichtung ist mithin ein "real 3D"-Fertigungsverfahren realisierbar und die Präpolymervorstufe in alle Raumrichtungen polymerisierbar. Die traditionellen Schichtungsphänomene werden hier neutralisiert.

Die Steuereinrichtung ist vorzugsweise dazu eingerichtet, insbesondere programmiert, alle für das Erzeugen der 3D-Struktur relevanten Betriebsparameter der Vorrichtung anhand von vorgegebenen CAD-Daten der 3D-Struktur zu steuern.

Besonders bevorzugt ist die Steuerungseinheit dazu eingerichtet, insbesondere programmiert, anhand einer repetitiven Bildgebung im Wege der
- Magnetresonanztomographie,
- der Computertomographie,
- der digitalen Volumentomographie,
- der Sonographie,
- des Laser-Scannings und/oder
- einer Positronen-Emissions-Tomographie
ermittelte Bilddaten der Polymervorstufe/der teilgefertigten 3D-Struktur mit den CAD/CAM Daten zu vergleichen und bei Überschreiten einer definierten Abweichung der Bilddaten von den CAD/CAM Daten die Betriebsparameter und/oder CAD/CAM-Daten für das Herstellen des übrigen 3D-Struktur derart anzupassen, dass (weiteren) Abweichungen bei der Fertigung des übrigen 3D-Struktur von den CAD/CAM Daten entgegengewirkt wird. Mit anderen Worten ist die Steuerungseinheit dazu eingerichtet, den Soll-/Ist Zustand der Polymerisation der Polymervorstufe mittels repetitiver Bildakquisen abzugleichen und die Steuerung des weiteren Herstellungsprozesses der 3D-Struktur auf Grundlage der so gewonnenen Daten fortzusetzen. Optimalerweise bietet sich hierzu die MRI an, obgleich vorgenannte alternative Bildgebungseinrichtungen - ggf. auch multimodal kombiniert - zum Einsatz kommen können.

Die erfindungsgemäße Vorrichtung ermöglicht somit insgesamt ein additives Fertigungsverfahren von 3D-Strukturen, das, basierend auf der Technik der Resonanz-Nischen Induktion, als **Magnet-Resonanz-Induktions-Polymerisation (= kurz: "MRiP"),** bezeichnet werden kann.

Das mittels der Vorrichtung fertigbare 3D-Struktur kann ein beliebiges Produkt aus einem Polymer- oder Polymerverbundwerkstoff sein. So kann die 3D-Struktur etwa ein Maschinenelement sein. Hier kommen insbesondere Achsen, Wellen, Lagerelemente, Getriebeteile, Dichtungselemente, Verbindungselemente, Gehäuse(teile) usw. in Betracht. Die 3D-Struktur kann auch ein Medizinprodukt, beispielsweise eine Epithese, eine Orthese, eine Bandage, eine Zahnspange, eine Zahnverblendung, ein Beatmungsschlauch, ein Gewebekleber, ein medizinisches Implantat oder auch eine (bio-)artifizielle Struktur für den Gewebe- bzw. Organersatz sein. Darüber hinaus kann die 3D-Struktur ein Alltagsgegenstand, etwa Schmuck, ein Uhrengehäuse, ein Spielzeug, ein Tragebehältnis, Geschirr, Besteck, eine elektrisch isolierende oder elektrisch isolierende Schicht oder ganz allgemein eine Beschichtung einer beliebigen anderen Struktur sein.

Auf der Grundlage präzise gesteuerter Induktionsphänomene, moderner Materialwissenschaften und Bildgebungsmodalitäten, CAx, Nanotechnologie, Stammzellforschung und Entwicklungsbiologie kann die erfindungsgemäße Vorrichtung durch die damit ausführbare Magnet-Resonanz-Induktions-Polymerisation das Potential generativer 3D-Verfahren in den medizinischen Kontext des Tissue Engineering und - unter Nutzung der Magnetresonanz als zugleich diagnostische, navigatorische, supervidierende Instanz und generatives Moment - direkt in den lebendigen Körper überführen.

Die in-situ-(Bio-)Fabrikation bietet von Anfang an alle Vorteile der natürlichen Geweberegeneration innerhalb eines physiologischen, bioresponsiven Milieus und löst damit die Kardinalprobleme herkömmlicher Gewebeersatzprodukte und konventioneller Bioreaktoren, wie mangelnde Stabilität, mangelnde Integrativität, mangelnde Adaptivität, mangelnde Interaktivität und unzureichende Vitalität. Hieraus folgt, dass eine präzise Stimulation der paramagnetischen Substanz über die Gestaltung der Binnenstruktur und Oberflächentextur der Polymer-3D-Struktur direkten und indirekt Einfluss auf die Qualität und Intensität dessen Wechselwirkungen mit dem Empfängerorganismus nimmt. Wer also die elektromagnetische Induktion beherrscht, beherrscht auch die biomimetische Induktion und dadurch die bioaktive Kompetenz des Implantates und die bioauthentische Kapazität des Regenerates.

Die erfindungsgemäße Vorrichtung birgt den Schlüssel zur Universalität und das enorme Potential, eine 3D-Struktur herzustellen, die sowohl auf mikrotopographischer als auch makroarchitektonischer Ebene hochflexibel und individualisiert der Diversität therapiebedürftiger Gewebedefekte und -Rezipienten gerecht wird. Mittels der Vorrichtung kann eine 3D-Struktur hergestellt werden, die die natürliche Anisotropie hierarchisch organisierter biologischer Gewebe und deterministische Komplexität bioartifizieller Interfaces authentisch nachbildet, um zum frühestmöglichen Zeitpunkt und nachhaltig die biomimetischen Grundlagen für eine langfristige Funktionalität des Implantates im systemischen Gesamtverbund zu schaffen.

Die Vorrichtung kann beispielsweise beim in-vivo Herstellen der 3D-Struktur eingesetzt werden. Hier kann eine nahtlose Verknüpfung der zu erzeugenden 3D-Struktur mit köpereigenen oder körperfremden Strukturen, d.h. deren Verankerung am Zielort mit dem jeweils umliegenden Gewebe, realisiert werden. Damit erlaubt die Vorrichtung ein direktes in-situ-3D-Bioprinting im lebenden Organismus. Die optionale Vitalisierung der 3D-Struktur durch passive und/oder aktive Zellbesiedelung kann beispielsweise kontaktlos und minimalinvasiv realisiert werden.

Weist die Vorrichtung zumindest eines oder auch mehrere der vorstehend genannten Einrichtungen für die Bild(daten)akquise auf, wird dadurch eine bildgesteuerte bzw. bildnavigierte Applikation der Polymervorstufe am vorgegebenen Zielort ermöglicht. Dies ist insbesondere bei der in-vivo Fertigung des 3D-Struktur von Vorteil.

Zu beachten ist, dass die erfindungsgemäße Vorrichtung bei seiner Verwendung im medizinischen Bereich im weiteren Sinne eine 8D-Fertigung der 3D-Struktur ermöglicht. Also einer Technologie, die achsenfrei in allen Raumrichtungen Material "addieren" kann ("real" 3D). Die 3D-Struktur kann bei der in-vivo Fertigung mit ihrer Umgebung interagieren (4D), diese instruieren (5D), durch Zellen vitalisiert werden (6D) und hierdurch wandlungsfähig bis zur vollkommenen biologischen Integration (7D) sein, darüber hinaus aber auch kontaktlos und ggf. mehrzeitig von extern modifiziert werden (8D). Besonders hervorzuheben ist hierbei, dass die "real" 3D-Prozessierung eine homogene isotrope - also uniforme - Belastbarkeit der 3D-Struktur gewährleistet, wohingegen klassische 3D-Druck-Produkte abhängig von der traditionellen Aufbauachse (z-Achse) in der Regel ein richtungsabhängiges mechanisches Lastaufnahmevermögen aufweisen.

Im medizinischen Kontext dient die erfindungsgemäße Vorrichtung bzw. das erfindungsgemäße 3D-Verfahren MRiP dem Ziel, die Integrität und Interaktivität funktioneller anatomischer Gesamtgefüge zu reparieren, zu rekonstruieren, zu respektieren, zu korrigieren und zu optimieren, um authentische regenerative Vorgänge zu stimulieren und zu amplifizieren. Dies gilt insbesondere für sehr kleine anatomische Funktionseinheiten und sehr große Gewebevolumina, welche sich bislang einer suffizienten "Restitution ad integrum" mit herkömmlichen Techniken entzogen.

Mittels der erfindungsgemäßen Vorrichtung /des erfindungsgemäßen Systems ist eine in-vivo Fertigung der 3D-Struktur auch bei all jenen Patientenkollektiven, deren Risikoprofil bislang invasive Therapieverfahren einschränkte oder nicht erlaubte, möglich.

Das Prinzip der kontaktlosen Wärmeerzeugung durch induktive Leistungsübertragung mittels elektromagnetischer Wechselfelder bzw. HF-Strahlung ist allgemein bekannt und findet abseits industrieller Herstellungsprozesse seit Jahrzehnten Anwendung in der physikalischen Medizin. Dass sich durch Kopplung magnetischer Nanopartikel an elektromagnetische Wechselfelder im menschlichen Körper biologisch wirksame und reliabel therapeutisch nutzbare Wärmephänomene erzeugen lassen, belegt nicht zuletzt auch die FDA-Zulassung der onkologischen interstitiellen Hyperthermie.

Eine Instrumentalisierung dieser Methode zum additiven Strukturaufbau im Sinne einer induktiven Polymerisation thermosensibler Polymer-Komposit-Präparationen zu mehrdimensionalen Strukturen wurde jedoch bislang nie beschrieben. Dies mag der Tatsache geschuldet sein, dass eine hochpräzise Fokussierung elektromagnetischer Felder bis heute selbst unter Laborbedingungen nur mit unverhältnismäßig großem technischem Aufwand gelingt. Eine grobe Fokussierung, wie sie sich z.B. mittels frequenzkohärenten Verstärkern erreichen lässt, ist für präzise additive Technologien hingegen nicht ausreichend.

Da die elektromagnetische Induktion jedoch im Gegensatz zu den etablierten traditionellen Verfahren der kontaktlosen Energieübertragung (UV-, IR-, Ultraschall, LASER) weder mit zunehmender Laufstrecke (Eindringtiefe) noch an Gewebeübergängen relevante unerwünschte Interferenz- und Absorptionsphänomene zeigt und unter Einhaltung gesetzlicher Dosisgrenzwerte und Frequenzspektren kein biologisch schädliches Potenzial aufweist, keiner Schutzatmosphäre oder starrer, mechanischer Führungssysteme bedarf, ist sie als idealer Energievermittler für die kontaktlose 3D-Fertigung, insbesondere auch bei der in-situ-Biofabrikation, zu betrachten. Die außergewöhnliche Universalität von MRiP ergibt sich dabei aus der ihr eigenen multiparametrischen Strategie, Strukturen aus kleinstmöglichen Untereinheiten aufzubauen, resultierend in einer maximalen Gestaltungsfreiheit und Adaptabilität.

Die erfindungsgemäße Vorrichtung, das System und auch das Verfahren (=MRiP) zum Erzeugen der 3D-Struktur bieten nun erstmals einen praktikablen Lösungsansatz, wie induktive Energiedepositionen auch mittels ungerichteter elektromagnetischer Wechselfelder (HF-Feld) ortsspezifisch realisiert, moduliert und gezielt zum kontrollierten additiven Strukturaufbau genutzt werden können.

Im natürlichen Zustand weisen alle ausreichend kleinen paramagnetischen Substanzen, insbesondere superparamagnetische Nanopartikel, etwa nanopartikuläre Magnetitpartikel, im Sinne von Nano-Oszillatoren ein durch ihr elektromagnetisches Umgebungsmilieu und ihren individuellen Energiegehalt definiertes jeweils eigenes minimales Schwingungsmoment auf. In einem homogenen statischen Magnetfeld, dessen Feldstärke ein Vielfaches der elektromagnetischen Umgebungsmilieus der Oszillatoren beträgt, richten alle Oszillatoren ihre Rotationsmomente parallel bzw. antiparallel zu den Magnetfeldlinien des statischen Feldes aus und sind durch ein äußeres HF-Feld mit einer Frequenz, die der Resonanzfrequenz f₀ der paramagnetischen Oszillatoren entspricht oder im Wesentlichen entspricht, zu thermogenen Oszillationen anregbar.

Die Steilheit und Schnelligkeit der **dynamischen Gradientenfelder** der Vorrichtung ist in mind. 3 Raumrichtungen vorgebbar, um die jeweilige Voxel-Dimension (räumlich/zeitlich) und die Voxel-Verteilung (räumlich/zeitlich) zu definieren.

Nach der Erfindung kann der Arbeitsbereich der Vorrichtung innerhalb einer Umhausung bzw. eines Gehäuses der Vorrichtung angeordnet sein. Dadurch kann eine definierte Arbeitsumgebung für das Erzeugen der 3D-Struktur bereitgestellt und aufrechterhalten werden. So können beispielsweise die Temperatur, die Zusammensetzung der Atmosphäre (=Arbeitsatmosphäre), der Atmosphärendruck im Arbeitsbereich sowie auch die Feuchtigkeit der den Arbeitsbereich unmittelbar umgebenden Arbeitsatmosphäre vereinfacht und kostengünstig dem Bedarf entsprechend eingestellt werden. Die Umhausung kann aus Kunststoff, beispielsweise in Form einer Kunststofffolie, aus Glas oder aus einem anderen MRI-geeigneten Material bestehen.

Besonders bevorzugt weist die Vorrichtung eine Pumpe auf, mittels derer der Arbeitsbereich mit einer für den jeweiligen Fertigungsprozess vorgegebenen Arbeitsatmosphäre befüllbar bzw. im Arbeitsbereich ein subatmosphärischer Druck bzw. ein näherungsweises Vakuum aufbaubar ist.

Die Vorrichtung kann nach der Erfindung eine Temperiereinrichtung zum Temperieren des Arbeitsbereichs bzw. der im Arbeitsbereich angeordneten Polymervorstufe aufweisen. Mittels der Temperiereinrichtung kann die Polymervorstufe bedarfsweise gekühlt werden, um z. B. vorab und/oder während der Herstellung der 3D-Struktur einer unerwünschten unkontrollierten Polymerisation der Polymervorstufe außerhalb des zur Polymerisation ausgewählten/bestimmten Voxels entgegenzuwirken. Mittels der Temperiereinrichtung kann der Arbeitsbereich bzw. die darin angeordnete Polymervorstufe bei Bedarf aber auch aufgeheizt, d. h. "angelassen", werden, um deren Polymerisation zu begünstigen.

### Polymervorstufe

Die Erfindung bezieht sich auch auf eine Polymervorstufe mit zumindest einer paramagnetischen Substanz zum Fertigen (=Erzeugen) einer 3D-Struktur.

Die Polymervorstufe umfasst nach der Erfindung Monomere und/oder Oligomere und/oder Polymere, die im Wege einer thermischen Polymerisation, d. h. durch Einwirken von thermischer Energie, polymerisieren.

Die Polymervorstufe kann insbesondere sogenannte Biopolymere umfassen. Diese zeichnen sich durch eine hohe Biokompatibilität, hohe Bioaktivität und Zellbindungskapazität aus. Nach der Erfindung kommen hier beispielsweise Polysaccharide, Glykosaminoglykane, Polypeptide und/oder Proteine in Betracht. Insbesondere Alginate, Hyaluron, Kollagene/Gelatine, Chitosan, Fibrin, Seidenfibroin, Cellulose und selbst Derivate der Extrazellulärmatrix des Menschen (ECM-Derivate) und sogenannte (bio-)artifiziellen Polymere sind vorstellbar. Auch marine Kollagene, etwa aus Fischabfällen (Fisch-Gelatine-Metacrolyl = FGelMa), sind vorstellbar.

Artifizielle Polymere wiederum weisen eine hohe mechanische Stabilität und präzise modulierbare Eigenschaften (z.B. definierte Abbaugeschwindigkeit) auf. Hierzu zählen traditionelle Werkstoffe der Biotechnologie + Pharmakologie, aber auch in zunehmendem Maße etablierte Substanzen der kunststoffverarbeitenden Industrie, von denen nachstehend einige geeignete genannt sind:
PLA (Poly-Milchsäure), PEG (Polyethylenglykol), PCL (Polycaprolacton; ein sehr guter Ausgangsstoff für anorganische Biokeramiken), PGA (Polyglykolsäure), PLGA (Poly(-lactid-co-glycolid), PEO (Polyethylenglycol), PPO (Polyphenylenoxid), PU (Polyurethan), PEEK (Polyetheretherketon), Polyamide (Nylon; +/- Polyester), PCU-Sil (Polycarbonat-basierte Urethan-Silikone), PUU (Poly-Urea-Urethane), SMP (shape-memory-Polymere), Acrylnitrile (z.B. ABS: Acrylonitril-Butadien-Styrol), Block-Copolymere, Flüssig-Kristall-Polymere.

Um die Vorteile beider Gruppen zu nutzen, kann die Polymervorstufe nach der Erfindung einen Multi-Material-Mix (z.B. PEG-Kollagen-Hydrogele) umfassen.

Die paramagnetische Substanz umfasst vorzugsweise paramagnetische Partikel, insbesondere in Form paramagnetischer Mikro- oder Nanopartikel. Die paramagnetische Substanz ist vorzugsweise hochspezifisch frequenzselektiv, d. h. durch Einstrahlung eines HF-Feldes mit definierter Frequenz bzw. einem definierten Frequenzband zu Oszillationsbewegungen anregbar. Durch diese Oszillationsbewegungen der paramagnetischen Substanz kann ein thermogener Energieeintrag in die Polymervorstufe zur Polymerisation der Polymervorstufe erreicht werden.

Die Konzentration der vorgenannten Partikel beträgt vorzugsweise > 1000 Partikel pro Milliliter der Polymervorstufe, insbesondere > 10.000 Partikel pro Milliliter der Polymervorstufe. Dadurch ist während des Fertigungsprozesses eine zuverlässige und homogene Polymerisation der Polymervorstufe im jeweilig ortskodierten Voxel der Polymervorstufe gewährleistet. Die Konzentration der Partikel kann in Abhängigkeit von der aus der Polymervorstufe zu erzeugenden 3D-Struktur bzw. der Zusammensetzung der Polymervorstufe dem Bedarf entsprechend eingestellt sein. Nach der Erfindung kann die Konzentration der Partikel bis zu 10¹⁷ Partikel pro Milliliter der Polymervorstufe betragen. Dadurch können auch kleinste 3D-Struktur und mit einer bislang unerreichten Detailauflösung additiv gefertigt werden.

Die paramagnetische Substanz umfasst nach der Erfindung vorzugsweise Metallpartikel. Die Partikel können beispielsweise aus Magnetit (Fe₃O₄) oder einem Silberhalogenid (AgₙXₙ) bestehen.

Die paramagnetische Substanz ist nach einer Ausführungsform der Erfindung vorzugsweise in der Polymervorstufe suspendiert. Eine möglichst homogene Verteilung/Suspension der paramagnetischen Partikel in der Polymervorstufe ist vorteilhaft.

Nach einer alternativen Weiterbildung der Erfindung kann die paramagnetische Substanz auch zumindest teilweise oder vollständig an Monomere/Polymere der Polymervorstufe gebunden sein. Derlei Metallorganyle bzw. metallorganische Verbindungen weisen in der Regel eine polare kovalente Bindung zwischen einem Kohlenstoff-Atom und mindestens einem Metall- oder elektropositiven Elementatom auf.

Nach einer besonders bevorzugten Weiterbildung der Erfindung können sich die paramagnetischen Partikel der Polymervorstufe zumindest teilweise in ihren Materialeigenschaften bzw. in ihrer spezifischen chemischen Zusammensetzung und/oder Größe voneinander unterscheiden.

Im Hinblick auf die Biokompatibilität der Partikel können die Partikel jeweils eine Beschichtung aufweisen. Die Beschichtung der Partikel kann beispielweise Titan umfassen. Denkbar sind auch andere biokompatible Beschichtungsmaterialien, wie etwa Polyetheretherketon (PEEK), Polyetherimid (PEI), Polycarbonate, AcrylnitrilButadien-Styrol, Polylactide (PLA), Polyhydroxyessigsäure, Polyglycolsäure.

Die Polymervorstufe kann darüber hinaus (zusätzlich zur paramagnetischen erfindungsgemäß einen oder mehrere Zuschlagstoffe (=Additiva) umfassen. Die Zuschlagstoffe können organische und/oder anorganische Zuschlagstoffe sein.

Die Kapazität optionaler biologischer, chemischer, physikalischer und pharmazeutischer Additiva sollte keinesfalls unterschätzt werden. Diese dienen beispielsweise als Induktoren, Promotoren, Katalysatoren und Terminatoren der (bio-)chemischen Reaktivität, der Homogenisierung und Stabilisierung des Milieus bzw. der Resonanzbedingungen und somit auch der Artefaktreduktion. Gleichermaßen können sie zu einer Erhöhung als auch zur Minderung der elektrischen bzw. thermischen Leitfähigkeit bzw. Isolierung innerhalb der Polymervorstufe führen und hierdurch die Polymerisation positiv oder negativ beeinflussen, sensible internale und externale Zonen protegieren und ggf. das Post-Processing vereinfachen.

Insbesondere Nanopartikeln gelingt es in kleinesten Mengen die originären Eigenschaften von Werkstoffen und ihren Endprodukten dramatisch zu verändern, diese zu funktionalisieren und zu (bio-)aktivieren (z.B. Au, Ag, Montomorillonite, Laponite, Hectorite, Silica, Fe2O3, Fe3O4, Graphene, GraphenOxide, Nanocellulose, LDHs (layered double hydroxides, Pyrrole, ...).

Die Additiva können nach einer Weiterbildung als Adsorbantien fungieren, sei es zur Bindung oder Umwandlung toxischer oder kontraproduktiver Meta- und Kataboliten, unerwünschter Botenstoffe oder Schmerzmediatoren, um z.B. immunologische Abwehr- und Sensibilisierungskaskaden oder Infektionen zu verhindern.

Geeignete Additiva können es erlauben, die rheologischen Eigenschaften, u.a. die Oberflächenspannung der Polymervorstufe, zu beeinflussen, um deren Injektabilität und Mikroadhäsivität zu maximieren.

Andere geeignete Additiva wiederum können helfen, die Endkonsistenz der 3D-Struktur gezielt zu modulieren bzw. zu limitieren (z.B. Füllstoffe). Gleichermaßen erwähnenswert sind Zuschlagstoffe in Form von Biologika zur Zellaktivierung und Steuerung wie Wachstumsfaktoren und Immunmodulatoren (z.B. Interferone), Radiomodulatoren zur Unterstützung von Strahlentherapien und Zytostatika, aber auch Visualisierungshilfen wie Kontrastmittel oder andere kontaktlos von extern detektierbare und quantifizierbare Marker.

Die Zuschlagstoffe können mithin insbesondere aus der Gruppe der
- Fasern,
- Farbstoffe,
- antibakteriellen Substanzen,
- Wachstumsfaktoren
- Nanopartikel/-tubes,
- mineralischen Füllstoffe (z.B. Tricalcium-Phosphat-Zement, Nano-Hydroxyapatit, bioactive glass),
- metallischen Werkstoffe (z.B. Silber, Gold, Magnetite (Fe2O3, Fe3O4) - z.B. SPION = superparamagnetische Eisenoxidnanopartikel, Gd-Chelate/Konjugate),
- Glykosaminoglykane,
- sogenannten MMC-Stoffe (zum sogenannten macromolecular crowding; z.B. Dextrane oder Ficol (= Saccharose-Epichorhydrin-Copolymer))
- Polypeptid-Motive wie -RGD-Sequenzen (Arginin, Glycin und Asparaginsäure), die beispielweise in Proteinen der extrazellulären Matrix vorkommen (z.B. in Fibronectin und Vitronektin),
- -IKVAV + - YIGSR (Laminin) und
- Promotoren
- Terminatoren,
- Inhibitoren,
- Katalysatoren,
- Sensitizer,
- Immunmodulatoren (wie VGF oder das Molekül JNK3)
sein.

Die vorgenannten Zuschlagstoffe können frei gelöst, substanzgebunden, oder temporär fixiert an Transportmoleküle bzw. in Thermosomen vorliegen.

Die Polymervorstufe kann insbesondere eine liquide Form oder Gelform aufweisen. Besonders bevorzugt weist die Polymervorstufe eine Viskosität auf, die zwischen 10² und 10⁶ mPa·s beträgt.

Nach der Erfindung kann die Polymervorstufe auch in Form eines sogenannten "Squids" vorliegen. Derlei Squids sind anderweitig präformierte, unvollständig ausgehärtete Polymervorstufen.

Nach einer bevorzugten Weiterbildung der Erfindung ist die Polymervorstufe für den Menschen nicht-toxisch. Dadurch kann die Polymervorstufe im menschlichen Körper für die in-vivo-3D-Fertigung eingesetzt werden.

Ganz besonders bevorzugt ist die Polymervorstufe im nicht-auspolymerisierten Zustand im menschlichen und/oder tierischen Körper, beispielsweise durch körpereigene Enzyme, abbaubar.

### System

Das erfindungsgemäße System umfasst eine vorstehend erläuterte Vorrichtung zum Erzeugen eines 3D-Struktur sowie die vorstehend erläuterte Polymervorstufe mit zumindest einer paramagnetischen Substanz, insbesondere superparamagnetischen Metall- bzw. Magnetitpartikeln.

### MRiP-Verfahren

Das erfindungsgemäße Verfahren zum Erzeugen der 3D-Struktur weist die folgenden Schritte auf:
a. Definieren (102) von CAD/CAM-Daten (40) zu der zu fertigenden 3D-Struktur (30);
b. Bereitstellen (104) einer Polymervorstufe (28), die eine, bevorzugt homogen verteilte, paramagnetische Substanz umfasst;
c. Einbringen (106) der Polymervorstufe (28) in den Arbeitsbereich der Vorrichtung (12);
d. Ortskodieren (108) eines Voxels V innerhalb der Polymervorstufe (28) in Abhängigkeit von den CAD/CAM Daten (40) durch Anlegen magnetischer Gradientenfelder;
e. Polymerisieren (110) der Polymervorstufe (28) in dem zumindest einen ortskodierten Voxel V durch Einstrahlen (112) von HF-Strahlung (42) mittels derer die paramagnetische Substanz 32 in dem jeweiligen weiteren Voxel V zu Oszillationen angeregt wird; und
f. Nachfolgend sequentielles Ortskodieren (108) weiterer, bevorzugt räumlich jeweils aneinander angrenzender, Voxel V in der Polymervorstufe (28) in Abhängigkeit von den CAD/CAM-Daten (40) und Polymerisieren (110) der jeweilig weiteren ortscodierten Voxel V durch Einstrahlen (112) von HF-Strahlung (42), mittels derer die paramagnetische Substanz (32) in dem jeweiligen weiteren Voxel V zu Oszillationen angeregt wird.

Bei dem erfindungsgemäßen additiven Fertigungsverfahren für die 3D-Struktur beruht die Ortsauflösung auf den induktiven Magnetresonanzphänomenen eines Wechselspiels aus elektromagnetischer Resonanzisolation und Resonanzstimulation. Zwar nutzen diagnostische bzw. analytische MR-Verfahren diese Strategie. Allerdings wurden bislang keine spezifisch resonanzfrequenzselektiven, thermogenen, paramagnetischen Nanopartikel eingesetzt, die durch HF-Anregung in eine thermogene Oszillation versetzt werden und so eine lokal auf das ortskodierte Voxel der Polymervorstufe begrenzte Polymerisierung der Polymervorstufe, d.h. Induktion chemischer Bindungen in der thermosensitiven Polymervorstufe, induzieren.

Das hier vorgeschlagene additive Fertigungsverfahren erlaubt eine kontinuierliche Volumen-Graduierung der Polymerisation (kleiner, gleich und größer einer herkömmlichen Schichtdimension) der Polymervorstufe, also eine sowohl hochpräzise punktuelle als auch "en bloc" Polymerisation und damit Aushärten der zu erzeugenden 3D-Struktur.

Damit wird ein effizientes 3D-Fertigen beliebiger, insbesondere bio-artifizieller Strukturen, sowie deren Modifikation ermöglicht. Die präzise HF-Stimulation frequenzselektiver thermogener Nanooszillatoren in elektromagnetischen Resonanznischen, welche ortsspezifisch, vorzugsweise auch auf der Basis hochauflösender Bilddaten, durch gezielte Überlagerung magnetischer Gradientenfelder innerhalb einer mehr oder weniger liquiden thermosensitiven Polymervorstufe erzeugt werden, ermöglicht wie bereits dargelegt, die Fertigung von 3D-Strukturen jedweder Geometrie, Konfiguration und Komplexität - in Abhängigkeit der eingesetzten Materialien auch jeglicher Konsistenz - individuell in vitro und auch in vivo. Die Strategie, Strukturen aus kleinstmöglichen Untereinheiten aufzubauen, resultiert in einer maximalen Gestaltungsfreiheit und Adaptabilität des Verfahrens.

Dies in einer bis dato unerreichten Detailtreue und Geschwindigkeit. Das Verfahren kann für die Fertigung von Maschinenelementen, medizinischen Produkten, insbesondere bei der medizinischen und biotechnologischen Biofabrikation, und sogar beim in-situ-Bioprinting neue Wege ermöglichen. Dies insbesondere auch deshalb, weil das erfindungsgemäße Verfahren (MRiP) eine präzise Oberflächengestaltung und eine nahtlose Verknüpfung bzw. Verankerung mit anderen Strukturen ermöglicht. Dadurch kann bei Implantation der 3D-Struktur eine vereinfachte und zuverlässigere Vitalisierung durch passive/aktive Zellbesiedelung begünstigt werden. Im medizinischen Kontext kann MRiP ermöglichen, Implantate zu erzeugen, die die Integrität und Interaktivität funktioneller anatomischer Gesamtgefüge reparieren, rekonstruieren, respektieren, korrigieren und optimieren, um natürliche regenerative Vorgänge zu stimulieren und zu amplifizieren.

Eine grobe Fokussierung, wie sie sich z.B. mittels frequenzkohärenten Verstärkern erreichen lässt, ist für präzise additive Technologien nicht ausreichend. Die elektromagnetische Induktion zeigt im Gegensatz zu den etablierten traditionellen Verfahren der kontaktlosen Energieübertragung mittels UV-/IR-Strahlung, Ultraschall oder LASER weder mit zunehmender Laufstrecke in der Polymervorstufe bzw. im Gewebe (Eindringtiefe) noch an Materialübergängen relevante unerwünschte Interferenz- und Absorptionsphänomene. Darüber hinaus weist die elektromagnetische Induktion unter Einhaltung gesetzlicher Dosisgrenzwerte und Frequenzspektren kein biologisch schädliches Potenzial auf, bedarf per se keiner Schutzatmosphäre oder starrer, mechanischer Führungssysteme und ist deshalb als ein idealer Energievermittler für die kontaktlose in vitro sowie auch in-vivo-Biofabrikation zu betrachten.

Das erfindungsgemäße MRiP-Verfahren bietet nun erstmals einen praktikablen Lösungsansatz, wie induktive Energiedepositionen mittels ungerichteter elektromagnetischer Wechselfelder (HF-Feld) ortsspezifisch realisiert, moduliert und gezielt zum kontrollierten additiven Strukturaufbau genutzt werden können.

Im natürlichen Zustand weisen alle superparamagnetischen Oszillatoren in einer (standardisierten) thermosensitiven Polymervorstufe, z. B. eines Polymer-Komposit-Gels, ein durch ihr elektromagnetisches Umgebungsmilieu und ihren individuellen Energiegehalt definiertes jeweils eigenes minimales Schwingungsmoment auf.

In einem homogenen statischen Magnetfeld, dessen Feldstärke ein Vielfaches der elektromagnetischen Umgebungsmilieus der paramagnetischen Oszillatoren beträgt, richten alle Oszillatoren ihre Rotationsmomente parallel bzw. antiparallel zu den Magnetfeldlinien des statischen B₀-Felds aus, einhergehend mit einer Synchronisierung ihrer Resonanzfrequenzempfänglichkeit für Wechselfeld-Impulse, welche - neben Material, Polarität und Konfiguration der Oszillatoren - proportional von der Feldstärke des homogenen Magnetfelds abhängt. Dies bedeutet, dass für jede Feldstärke optimalerweise nur eine einzige, spezifische Frequenz existiert, welche alle entsprechend konfektionierten Oszillatoren maximal stimuliert - die sogenannte Resonanzfrequenz. Unter Resonanzbedingungen weisen also alle empfänglichen Oszillatoren eine maximale Energieabsorptionsbereitschaft hinsichtlich des spezifischen Hochfrequenz-Wechselfeldes auf, resultierend in einer signifikant erhöhten Oszillationsbewegung, welche aufgrund von Widerstands- und Reibungseffekten mit minimalem Kopplungsabstand in thermische Energie umgewandelt wird. Wie in der Kernspintomographie zur Schichtselektion und Ortscodierung dienen MRiP dynamische Gradientenfelder in mindestens 3 Raumrichtungen zur "indirekten Fokussierung" dieser Hochfrequenz-Impulse. Diese Magnetfelder sind durch einen kontinuierlichen Anstieg bzw. Abfall der jeweiligen Magnetfeldstärke entlang ihrer charakteristischen Achsen [x, y, z], relativ zum statischen permanenten Magnetfeld und der im Arbeitsbereich den Magnetfeldern ausgesetzten Polymervorstufe gekennzeichnet.

Ein durch spezielle Spulen zugeschaltetes Gradientenfeld in der x-Achse überlagert also das zuvor homogene permanente statische B₀-Feld und führt somit zu einem linearen Anstieg bzw. Abfall der statischen Gesamtfeldstärke entlang der x-Achse. Soweit technisch realisierbar, wären steilere nicht-lineare Gradienten vorteilhaft, um die Differenzierung von Nachbar-Voxeln zu verstärken. Gleiches gilt für jeweils zugeschaltete Gradientenfelder entlang der y-Achse und z-Achse, mit der Folge, dass jedes definierte Voxel des Gesamtvolumens des Arbeitsbereichs und damit der Polymervorstufe bzw. theoretisch jeder Oszillator im dreidimensionalen Raum über seine individuelle, ihm eigene elektromagnetische Nische im Schnittpunkt der mindestens 3 Gradientenfelder verfügt.

Unterscheidet sich also das magnetische Mikromilieu jedes Oszillators - bzw. Voxels - in allen Raumrichtungen linear vom magnetischen Mikromilieu seines Nachbaroszillators -bzw. Voxels -, dann verfügt jeder Einzeloszillator - bzw. jedes Voxel - über seine ganz eigene individuelle Resonanzfrequenz und lässt sich folglich individuell isoliert adressieren und selektiv anregen. Hierbei definiert die Steilheit der Gradientenfelder die Kantenlängen der Voxel in allen 3 Raumrichtungen, optimalerweise durch simultane summarische Überlagerung ihrer lokalen Feldstärke, alternativ sequentiell, z.B. unter Verwendung der inversen Fourier-Transformation. Im Unterschied zu allen herkömmlichen additiven Verfahren lassen sich auf diese Weise entweder Einzel-Voxel als auch zusammenhängende Volumeneinheiten im Sinne eines multi-Voxel processings "en bloc" polymerisieren.

Anstelle einer einzigen externen Wärmequelle stellt also jeder Nano-Oszillator in sich eine autonome Wärmequelle (mit minimalem Kopplungsabstand) dar, deren Wirkung ohne adressierende Gradientenfelder in einem dreidimensionalen thermischen gitternetzförmigem Erwärmungsmuster aus zunächst multiplen isomorphen und isothermen Hitzeinseln mit Temperaturmaxima jeweils unmittelbar um jeden einzelnen Oszillator resultieren würde. Die charakteristische Leistungsaufnahme der Oszillatoren, ihr Wirkungsgrad und die materialspezifische Wärmeleitung der Polymervorstufe definieren in einem ansonsten geschlossenen, perfekten System die Dimension, Dynamik und Dauer der Wärmeausbreitung bis zu jenem Zeitpunkt, an welchem eine global homogene Erwärmung des Gesamtvolumens erreicht ist, abhängig von Impulsintensität und Impulsperiodik, welche fortwährend in Echtzeit vom Anwender gesteuert werden können.

In der Praxis führen Wärmeleitungsphänomene zu einem zentrifugalen Temperaturabfall um jeden stimulierten Oszillator, entsprechend einer thermodynamischen, von den Umgebungsbedingungen abhängigen Penumbra. Diese thermischen Inseln neigen dazu, mit zunehmender Anregung über die Zeit zu konfluieren, sich auszugleichen oder gegenseitig zu verstärken.

Unter proaktiver Nutzung der beschriebenen Gesetzmäßigkeiten und nachfolgend aufgeführten Einflussfaktoren können diese Effekte vom Anwender durch fokussierte Stimulation einzelner individueller Oszillatoren(gruppen) isoliert lokal verstärkt oder reduziert werden, um punktuell bzw. zonal die finalen Wärmemuster und konsekutiv die Detailschärfe und Zieleigenschaften des Produktes (z.B. Funktionalität, Haltbarkeit) zu modulieren.

Differenzen der Resonanzempfänglichkeit erlauben also durch kontrollierte Variation der räumlichen und zeitlichen Resonanzbedingungen eine gezielte ortsspezifische Anregung thermogener Nanooszillatoren (= paramagnetische Substanz) (in vitro wie in vivo) und folglich einen hochpräzisen generativen Aufbau komplexer 3D-Strukturen auf der Basis differenzierter Energieabsorptionsmuster und ortsspezifisch reproduzierbarer Temperaturprofile. Die thermischen Effekte elektromagnetisch induktiv stimulierter Nanooszillatoren erlauben dabei neben der Modulation der internalen Polymer-Kohäsion, insbesondere auch eine Steuerung der Adhäsion der 3D-Produkte durch mikroskopische Strukturverzahnung (Mikroadhäsion) an fremden Oberflächen - eine primär zweckmäßige Rheologie der Polymervorstufe vorausgesetzt.

Bei Implantaten können diese in der Übergangszone zum Organismus (Interface) ggf. durch diskontinuierliche thermokoagulatorische Klebeeffekte ergänzt werden, woraus sich optimaler Weise eine kontinuierliche, nahtlose regenerative Einheilung ergibt. In vivo kann dies durch postentzündliche reparative bzw. postnekrotische fokale, interkonnektive Narbenbildungen flankiert werden. Darüber hinaus können ortsspezifische induktive Effekte das Implantatbett - also den Wundgrund - zuvor in förderlicher Weise konditionieren, z.B. durch thermisches Mikrodebridement, Blutstillung und Denervierung.

Der besondere Reiz des erfindungsgemäßen Verfahrens liegt dabei in der subtilen, multiparametrischen Steuerbarkeit der schrittweisen Aufbauphasen der Produktstruktur in Echtzeit, resultierend in einer maximalen Prozesskontrolle, Individualisierbarkeit und Ergebnisqualität.

Grundvoraussetzung hierfür ist eine differenzierte prädiktive Kalkulation der in allen Raumrichtungen zu applizierenden Energiedosen zur temperaturabhängigen Materialsolidifizierung. Die Modulation der pro Volumeneinheit übertragenen Energiedosis gelingt hierbei vor allem durch die zielorientierte Anpassung der elektromagnetischen Induktionsparameter und temporospatiale algorithmische Variation der Resonanzbedingungen, welche sich aus dem Zusammenspiel des statischen mit den dynamischen Magnetfeldern ergibt.

Hierbei gelingt es ungerichteten HF-Stimulationsimpulsen - beispielhaft mit einer Wellenlänge im mehrstelligen Zentimeterbereich (MRT-Diagnostik) - problemlos, selektiv Voxel im Submillimeterbereich anzuregen, ohne relevante Schwächung der eingestrahlten elektromagnetischen Energie durch Laufstrecke, Grenzflächen oder Phasenübergänge.

Gemäß einer ersten Ausführungsform der Erfindung kann die ResonanzEmpfänglichkeit jedes individuellen Nano-Oszillators sequentiell durch eine gezielte Variation der Gradientensteilheit und -Stärke an eine immerzu gleichbleibende Anregungsfrequenz bei allzeit frequenzkonstantem Stimulationsimpuls angepasst werden.

Gemäß einer alternativen Ausführungsform kann die Frequenz des Stimulationsimpulses sequentiell gezielt variabel an eine durch allzeit konstante Gradienteneigenschaften gleichbleibende, individualisierte ResonanzEmpfänglichkeit eines jedes Voxels angepasst sein.

Während die erstgenannte Ausführungsvariante seit Jahrzehnten erfolgreich in der Ortscodierung der diagnostischen Kernspintomographie zum Einsatz kommt, finden Frequenzmodulationstechnologien Anwendung in der industriellen induktiven Metall- und Kunststoffverarbeitung, dies allerdings ohne den Einsatz adressierender Gradientenfelder. Die technische Ausführungsvariante 2 ist zum heutigen Zeitpunkt technisch noch sehr aufwändig.

Zum Erzeugen des B₀-Felds, das der Gleichrichtung der paramagnetischen Substanz d. h. der paramagnetischen Nano-Oszillatoren parallel bzw. antiparallel zu den Magnetfeldlinien sowie der Gleichschaltung ihrer feldstärkenabhängigen Empfänglichkeit für ihren charakteristischen, resonanzinduktiven Hochfrequenzimpuls dient, kann ein Permanentmagnet, ein supraleitender Magnet, ein Elektromagnet oder ein Widerstandsmagnet eingesetzt werden. Entsprechend kann die Vorrichtung einen in der vorstehenden Weise ausgeführten Magneten aufweisen. Die Feldstärke des (stratischen) B₀-Felds kann nach der Erfindung ≤ 3 Tesla oder auch ≥ 3 Tesla sein. Ganz besonders bevorzugt ist die Feldstärke des B₀-Feldes > 10 Tesla. Gleichwohl die Magnetresonanztomographie bezüglich der Bild(daten)gewinnung bzw. bildmorphologischen Überwachung des Fertigungsprozesses als Goldstandard anzusehen ist, kann die Feldstärke des B₀-Felds nach der Erfindung im mT-Bereich liegen oder größer sein.

Die Gradientenfelder zur ortscodierten Adressierung der paramagnetischen Substanz der einzelnen Voxel werden mittels Gradientenspulen in ≥ 3 Raumrichtungen generiert. Diese Gradientenfelder werden sequentiell kurzzeitig zugeschaltet, wobei deren Feldstärke entlang ihrer jeweiligen Raumachse kontinuierlich ansteigt bzw. abfällt.

Dadurch kann eine ortsspezifische Abschwächung/Verstärkung des statischen B0-Feldes und damit eine Ortscodierung der HF-Empfänglichkeit der paramagnetischen Substanz im dreidimensionalen Raum durch summarische Generierung magnetischer Mikromilieus (Resonanz-Nischen) erreicht werden. Grundsätzlich gilt, je größer die Anstiegsstärke und je kürzer die Anstiegszeit, desto besser die Detailauflösung des Polymerisationsprozesses. Sofern eine enbloc Polymerisation (also das Polymerisieren mehrerer Voxel bzw. eines größeren Volumes of interest) gewünscht ist, so sind flache Gradienten, also flache Anstiegsstärken vorteilhaft.

Der Hochfrequenz-Feldgenerator (=Hochfrequenz-Einheit) dient der Anregung der paramagnetischen Substanz oder Oszillatoren (unter Resonanzbedingung) mittels HF-Strahlung. Die Hochfrequenzeinheit kann dazu einen HF-Sendeverstärker und zumindest eine HF-Sendespule aufweisen. Die HF-Einheit kann in das Gehäuse der Vorrichtung integriert sein. Alternativ kann die HF-Sendespule in Form einer zum Arbeitsraum der Vorrichtung fei positionierbaren, d. h. mobilen, HF-Spule ausgeführt sein. Hier kommen beispielsweise eine anatomisch-ergonomische Oberflächenspule und eine intrakorporal platzierbare Spule (z. B. Endorektal-Spule oder eine Katheter-montierte Spule) in Betracht. Die Hochfrequenzeinheit kann erfindungsgemäß auch eine Mehrzahl von HF-Spulen in Form von Arrayspulen aufweisen.

Die Wirkdauer der HF-Strahlung ist so niedrig wie möglich "As low as reasonably achievable" (=ALARA) zu wählen, wobei die Ganzkörper SAR-Werte zu beachten sind.

Die Frequenz/Phasenlage/Amplitude der HF-Strahlung ist/wird erfindungsgemäß abgestimmt auf die charakteristische Resonanzfrequenz der anzuregenden superparamagnetischen Substanz bzw. Nanopartikel (Oszillatoren) bei gegebener Ziel-Feldstärke der überlagerten Magnetfelder (= statisches Magnetfeld + 3 Gradientenfelder) im jeweiligen Voxel. Ziel ist eine maximale induktive, maximal selektive Anregung der superparamagnetischen Substanz Nanopartikel (Oszillatoren) alleinig eines jeweilig adressierten Voxels unter Resonanzbedingungen.

Die Intervalle zwischen den Einstrahlungen des HF-Impulses (Impuls-Periodik) sind erfindungsgemäß in Abhängigkeit von den folgenden Faktoren definiert:
- Thermogenität (= charakteristische Leistungsaufnahme + thermischer Wirkungsgrad) jedes Einzeloszillators bzw. deren thermogene Summe/Voxel.
- thermische Transistionsschwelle(n) der Polymervorstufe
- Wärmeleitfähigkeit des Polymers bzw. seiner Vorstufen
- Polymerisationskinetik (materialspezifisch/architekturspezifisch)
- Polymerisationsmuster (s. CAD); z.B. Auflösungsgrad, Wärmebrücken/- akkumulierende Untereinheiten
   ∘ räumliche Impulsdichte
   ∘ zeitliche Impulsdichte
- (= Modulation des Energieeintrages über temporospatiale ImpulsAlgorithmik)
- ggf. Impulsamplitude/Einstrahlungswinkel bezüglich des anzuregenden Voxels

Die Intervallperiodik bzw. Impuls-Zug-Länge der HF-Stimulation wird primär von den thermodynamischen Effekten im vorgegebenen Setting definiert und ggf. limitiert von der maximalen Geschwindigkeit der Steuerungseinheit + Magnetfeldgradienten, um zwischen 2 präzisen 3D-Voxel-Resonanz-Isolationen ("indirekte Fokussierung") zu wechseln.

Nach der Erfindung
- kann prinzipiell nahezu jedes beliebige Intervall zwischen den Einzelimpulsen der HF-Strahlung liegen, sofern der voxelspezifische bzw. VOI-spezifische (volume of interest) Energieeintrag im Einzelnen bzw. als Summe jeweils den gewünschten thermischen Effekt im Voxel/VOI hervorruft;
- ist bei einer maximal schnellen Voxel- (bzw. VOI-) Resonanz-Isolation (Ziel-Codierung) theoretisch auch eine kontinuierliche HF-Strahlung (HF-Pulsation) möglich, da grundsätzlich nur jene Oszillatoren thermogen schwingen, für welche jeweilig Resonanzbedingungen herrschen;
- ist bei einer in-vivo Fertigung der 3D-Struktur eine fraktionierte ImpulsAlgorithmik, z.B. mit repetitiven HF-Strahlungs-Zyklen, möglich, die unter thermodynamischen Aspekten vorteilhaft ist und eine Minimierung der globalen HF- und damit Energieeinstrahlung in biologisches Gewebe ermöglicht.

Die Frequenz der in den Arbeitsbereich eingestrahlten HF-Strahlung kann nach der Erfindung grundsätzlich im Kiloherz bis Tetraherzbereich liegen. Besonders bevorzugt beträgt die HF-Frequenz 100 kHz oder 130 Khz bis zu 100 MHz.

Die Steuerungseinheit der Vorrichtung dient folgenden Aufgaben:
a. Anlagensteuerung/Überwachung
b. Datenmanagement
c. CAD-Einheit
d. Bildakquise/-Analyse/-Rekonstruktion

Es versteht sich, dass die Steuerungseinheit eine Applikationssoftware mit KI-Eigenschaften aufweisen kann.

Die Detailgüte der polymeren 3D-Struktur hängt dabei ab von
- den Eigenschaften der paramagnetischen Substanz/Partikel bzw. (super)paramagnetischen Nanooszillatoren, wie deren
   - Frequenzselektivität
   - charakteristische Leistungsaufnahme
   - thermischem Wirkungsgrad.

Diese Eigenschaften können für die jeweilige paramagnetische Substanz bzw. die Nano-Oszillatoren experimentell bestimmt werden.
- der Konzentration und dem Verteilungsmuster der paramagnetischen Substanz in der Polymervorstufe.
- der Steilheit und Schnelligkeit der mit den Gradientenspulen erzeugten dynamischen Gradientenfelder in den 3 Raumrichtungen;
   - Voxel-Dimension (räumlich/zeitlich)
   - Voxel-Verteilung (räumlich/zeitlich)
- Dem Hochfrequenzgenerator bezüglich der
- Spezifität und Homogenität der induktiven HF-Strahlung,
   - Lage
   - Breite
   - Amplitude
   - (frgl. Einstrahlwinkel a relativ zum Hauptmagnetfeld)
- räumlichen und zeitlichen Verteilung der HF-Strahlung (= der HF-Impulse) (Impulsalgorithmik)
- 3D-Wärmemuster
- Polymerisationskinetik (z.B. Transitionsschwellen)
- materialspezifische thermodynamische Phänomene
- architekturspezifische thermodynamische Phänomene

Zu berücksichtigen gilt zudem eine abnehmende Detailauflösung des Verfahrens von einer prozeduralen Ebene zur nächsten:
- konstruktives Auflösungsvermögen (CAD)
- elektromagnetisches (induktives) Auflösungsvermögen
- thermisches Auflösungsvermögen
- zeitliches Auflösungsvermögen
- polymeres Auflösungsvermögen
- strukturelles Auflösungsvermögen
- diagnostisches Auflösungsvermögen (Bildgebung, z.B. MRT)
- post-processing Auflösungsvermögen (verfahrensabhängig)

Im klinischen Einsatz bietet in diesem Zusammenhang die Übergangszone des Polymer-Komposits zum Umgebungsgewebe besondere Herausforderungen, da hier signifikante thermische Verluste durch gewebespezifische Wärmeableitungen, Absorptions- und Perfusionsphänomene auftreten - ggf. auch Bewegungsartefakte. Außerdem gilt es relative und absolute thermische Toleranzschwellen biologischer Gewebe zu respektieren.

Limitierender Faktor des Auflösungsvermögens der diagnostischen MRT wird letztlich immer das Signal-Rausch-Verhältnis, also die Zahl der angeregten Protonen bzw. die Summe ihrer auslesbaren, provozierten Echos pro Volumeneinheit relativ zum Umgebungsrauschen bleiben. Dies deshalb, weil die stimulativen Hochfrequenzimpulse der MRT - abgesehen von Vorsättigungsimpulsen - immer alle Protonen einer Schicht bzw. im gesamten präselektierten 3D-Untersuchungsvolumen anregen und die genaue Ortscodierung erst im Ausleseschritt erfolgt. Demgegenüber werden gemäß der Erfindung idealerweise ausschließlich nur jene Oszillatoren präselektierter (=Resonanz-isolierter oder Resonanz-vulnerabler) Voxel der Polymervorstufe spezifisch angeregt, die gemäß Bauplan als Teil der 3D-Struktur ausgehärtet werden soll. Die Gradientenfelder werden also gemäß der Erfindung primär prospektiv aktiviert, wohingegen konventionelle MRT-Sequenzen zunächst mittels nur 1 Gradientenfeld (meist z-Achse) Schicht-Orientierung und -Dicke innerhalb des Untersuchungsvolumens definieren und erst nach dem HF-Impuls zum Zeitpunkt der Echo-Auslese weitere Gradientenechos (meist x-, y-Achse) zur retrospektiven Ortscodierung zugeschaltet werden. Während repetitive Stimulationszyklen für die Analyse der kernspintomographischen Summenechos mittels Fourier-Transformation unerlässlich sind - und zu einem unerwünschten Anstieg der Energieabsorptionsdosen führen - moderiert MRiP den Energieeintrag gezielt im Rahmen der temporospatialen Impulsalgorithmik.

Im Allgemeinen sollte die Resonanzfrequenz der Oszillatoren im Herstellungsprozess bewusst ungleich der Protonenresonanzfrequenz gewählt werden, um heterotope Polymerisationen durch MRT-Kontrollaufnahmen zu vermeiden. Eine Ausnahme stellen gewisse präformierte "Squids" z. B. 3D-Strukturen in Form thermoresponsiver Stents/Cages/Cava-Filter, Coils/Occluder, Herzklappen, Gefäß-Endoprothesen, thermoresponsiver Knochenzement bzw. Gewebekleber dar.

Weiterentwicklungen des Verfahrens könnten darüber hinaus von einer Kombination unterschiedlicher Oszillator-Typen mit korrespondierender Frequenzvariation profitieren. Schließlich führen bekanntermaßen hohe Frequenzen zu einem steileren Temperaturanstieg in unmittelbarer Nähe zum Oszillator, niedrige Frequenzen hingegen zu einer sanften, homogenen, globalen Erwärmung der weiteren Umgebung. Entsprechende Effekte könnten sich jedoch auch durch Variation des Einstrahlwinkels a im monofrequenten Impuls-Setting ergeben.

Während sich morphologische Merkmale < 200 µm also bislang in der klinischen MRT-Routine-Diagnostik nicht suffizient abbilden lassen, wird es MRiP bzw. mit der erfindungsgemäßen Vorrichtung/dem erfindungsgemäßen System gelingen, auch um Potenzen kleinere Strukturen (z.B. Poren) additiv zu generieren, in einer für die Zellnavigation, Zellkontrolle und histogenetische Determination relevanten Größenordnung zur räumlichen wie zeitlichen Steuerung der Geweberegeneration.

Das Fertigungsergebnis der MRiP kann nicht zuletzt durch eine repetitive EchtZeit-Bildakquise, z.B. auf der Basis robuster MRT-Sequenzen, alternativ auch durch andere Bildgebungseinrichtungen/-verfahren vergleichbarer Ortsauflösung (z.B. Oberflächen-LASER-Scanner, DVT etc.) weiter verbessert werden.

### Verwendung der Vorrichtung/der Polymervorstufe/des Systems

Die vorstehend erläuterte Vorrichtung bzw. das System bzw. die Polymervorstufe können universell im Bereich der Technik und der Medizin eingesetzt werden. So können sie insbesondere zum Erzeugen eines medizinischen Implantats, insbesondere von Knochenersatz, eines Traggerüsts für ein Organ oder Gewebe, oder einer Gefäßprothese eingesetzt bzw. verwendet werden.

Im Einzelnen:
Einsatz in der Human-, Zahn- und Tiermedizin:

### Plastische Radiologie:

- Stabilisierung von Gewebe/ das Erzeugen von Platzhaltern
- Rekonstruktion von Gewebe/den Ersatz von Gewebe
- Adaptation/ Verankerung/Embolisierung
- Augmentation/Conturing/Enhancement
- Kompartimentierung/Enkapselung, etwa pathologischer Prozesse, /Maskierung

Subtile Initialschäden können zum frühestmöglichen Zeitpunkt eingedämmt bzw. behoben und vulnerable individuell besonders gefährdete Gewebe a priori augmentiert werden, mit dem Potential Verschleißerscheinungen - z.B. im Leistungssport - und somit u.a. berufsbedingten Frühinvaliditäten systematisch vorzubeugen, resultierend in einem enormen gesundheitsökonomischen und volkswirtschaftlichen Benefit.

### Reparative & Rekonstruktive Radiologie:

Neben dem konkreten Ersatz von Binde- und Stützgeweben (Knorpel, Knochen, Sehnen, Bänder), eignet sich die Vorrichtung/die Polymervorstufe/das System sowohl zur plastischen Rekonstruktion, funktionellen und ästhetischen Formgebung und Formkorrektur als auch zur diffusen Gewebestabilisierung bei primär und sekundär herabgesetztem Gewebetonus.

Die Polymervorstufe bzw. die daraus erzeugte 3D-Struktur kann darüber hinaus als Bindemittel in Frakturzonen und Arthrodesen, als Klebstoff für die Versorgung akuter und als bioaktives Protektorat chronischer Wunden, als Netzersatz bei der Hernioplastik und als Platzhalter, Leitstruktur und Trägermaterial für zelluläre Strukturen und/oder azellulärer Additive und Wirkstoffe verwendet werden.

Darüber hinaus erlaubt die Erfindung die Synthese, Anastomosierung, Stabilisierung, Adaptation und Okklusion von Hohlräumen in vivo, einschließlich Klappen-, Sphinkter- und Shuntsystemen, weitgehend unabhängig von deren Dimension, Konfiguration und Lage. Dies verspricht erstmals für eine Vielzahl bislang nur unbefriedigend therapiebarer chronischer Erkrankungen wie pAVK, Lymphödeme und die chronisch-venöse Insuffizienz individualisierte kurative Strategien, dürfte aber auch das Outcome klassischer (mikrovaskulärer) Lappenplastiken und Akren-Replantationen signifikant verbessern.

Auch kann die Erfindung dazu verwendet werden, die biomechanische Kopplung von Prothesen zu optimieren und eines fernen Tages sogar neuronale Leitstrukturen gezielt intracorporal zu rekonstruieren und zu verknüpfen.

Insbesondere aber erlaubt die Erfindung, das Interface zwischen unterschiedlichsten Geweben und Materialien in situ authentisch zu gestalten, um eine optimale Gefüge-Integrität, Krafteinleitung und -übertragung zu bewirken. Diese können zu jedem Zeitpunkt präzise moduliert und wiederholt an differierende individuelle Belastungsprofile und komplexe Bewegungsmuster jedes Patienten angepasst werden. Dies durch bildgesteuerte Echtzeitmodulation der Prozessparameter der Vorrichtung als auch durch nicht-invasives und invasives Postprocessing.

Die Erfindung kann zum schonenderen und zugleich effizienteren Verankern eines Implantats, insbesondere einer Gelenk-Endoprothese verwendet werden. Bei deren Verschleiß können die Vorrichtung sowie auch die Polymervorstufe in vivo zur Neubeschichtung der Endoprothese eingesetzt werden.

Auch kann die Erfindung verwendet werden, um in situ einen groß- bzw. vollflächigen - bioartifiziellen Knorpelersatz zu erzeugen.

### Captive Radiologie:

Die Vorrichtung/das System/die Polymervorstufe können zur Gefahrenabwehr und Komplikationskontrolle tumoröser und entzündlicher Erkrankungen durch Abschottung befallener anatomischer Kompartimente mittels der 3D-Struktur eingesetzt werden. Dadurch können derlei krankhafte Prozesse in einem isolierten "neoanatomischen" Raum z. B. chemo- bzw. immuntherapeutisch, radioonkologisch oder thermisch therapiert - oder palliativ eingedämmt werden.

Da die Metastasierungswahrscheinlichkeit mit zunehmender Tumoroberfläche zunimmt, dürfte auch schon eine inkomplette Ummantelung von Tumoren mittels der in vivo erzeugten 3D-Struktur mit einem relativen Prognose-Benefit einhergehen. Die artifizielle polymere Ummantelung kann zudem als Leitstruktur für eine Biopsie, als Orientierungshilfe im Rahmen der tumorchirurgischen Resektion, als solidifizierter Sicherheitsabstand und ganz allgemein als Distanzhalter bzw. als Schutzschild zu vulnerablen Strukturen dienen.

### Manufaktive Radiologie:

Die Vorrichtung/das System bzw. die Polymervorstufe können beim in-vivo-Erzeugen von 3D-Strukturen in Form von Führungselementen, anatomischen (z.B. elektrokonduktive) Guidewires und polymeren 3D-Schienennetzen, aber auch bioartifiziellen Sensortechnologien und Leitersystemen verwendet werden. Dadurch kann eine sich abzeichnende Automatisierung der medizinischen Therapie und Diagnostik, etwa durch intrakorporal eingesetzte (teil-) autonome Miniaturroboter, Soft-Roboter oder, insbesondere intrakorporal, tragbare elektronische Geräte, weiter vorangebracht werden.

Zu beachten ist, dass die erfindungsgemäße Vorrichtung/das System bzw. die Polymervorstufe roboterassistiert, zur telemedizinischen Remote-Control-Intervention (remote manufacturing), insbesondere unter Nutzung von CAD-Konstruktions-Bibliotheken, verwendet werden können, um auch an entlegenen Orten - z.B. ISS - oder in Krisensituationen bestehende Versorgungslücken zu schließen und kritische Infrastrukturen aufrechtzuerhalten.

Die Polymervorlage kann abhängig vom Situs der in vivo zu erzeugenden 3D-Struktur, vom Zugangsweg, rheologischen Eigenschaften oder Patientenvariablen über Kanülen, Katheter, Endoskope oder andere zweckmäßige Instrumente direkt in den Körper bzw. in den Zielort eingebracht werden, sei es als Infusion, Injektion oder Instillat.

Eine mögliche Variante stellt das Balloning dar, also das Vorlegen mindestens eines, ein- oder mehrlumigen - z.B. kathetergetragenen - Ballon-Expanders zur lokalen Gewebevordehnung, woraufhin die Magnet-Resonanz-Induktions-Polymerisation wahlweise mit direktem Gewebekontakt oder aber primär im Ballon-Lumen erfolgen kann - z.B. bei Substanzunverträglichkeiten, oder der Notwendigkeit einer begleitenden chemischen Oberflächenaushärtung des Produktes bzw. dessen forcierter thermischer Nachbehandlung oder Kühlung mittels Perfusionssystemen.

Nach der Erfindung können für den Fertigungsprozess Voxel in der Polymervorstufe definiert werden, die jeweils eine einheitliche Größe aufweisen oder die sich in ihrer Größe zumindest teilweise voneinander unterscheiden. Durch das Definieren unterschiedlich großer Voxel bzw. VOI'S (volume of interest) können selbst 3D-Strukturen mit komplexer Geometrie zügiger erzeugt werden.

Während des Fertigungsprozesses kann zumindest ein Teil der auf Grundlage der CAD/CAM-Daten definierten Voxel anhand einer, insbesondere magnetresonanztomografischen, Bildgebung erfassten Bilddaten in ihrer räumlichen Position im Arbeitsbereich, ihrer Größe und/oder in ihrer Geometrie für den Fertigungsprozess geändert werden. Dadurch kann die Fertigungstoleranz der 3D-Struktur nochmals weiter verbessert werden.

Nach einer Weiterbildung der Erfindung wird der Fertigungsprozess, bevorzugt intervallweise, unterbrochen, um Bilddaten aus dem Arbeitsbereich, insbesondere von bereits erzeugten 3D-Struktur bzw. dem an die 3D-Struktur angrenzenden Volumen der Polymervorstufe zu erheben. Die Bilddaten können dabei insbesondere in Abhängigkeit von den CAD/CAM Daten der zu fertigenden 3D-Struktur erhoben werden.

Die Bilddaten werden vorzugsweise mit den CAD-Daten verglichen und bei Feststellen einer Abweichung des bereits (teil-)gefertigten 3D-Struktur die CAD/CAM-Daten für das Erzeugen der übrigen 3D-Struktur auf Grundlage der Bilddaten geändert. Dadurch kann eine außergewöhnlich kleine Fertigungstoleranz der 3D-Struktur realisiert werden.

Die 3D-Struktur kann nach der Erfindung teilweise oder vollständig innerhalb eines Lebewesens erzeugt werden, um nach dessen Entnahme als Implantat zur Verfügung zu stehen oder um in situ seine Funktion zu erfüllen.

Bei der 3D-Struktur kann es sich erfindungsgemäß um ein Traggerüst für Gewebe, ein Organ, insbesondere einen Knochen, eine Niere, eine Leber, einen Knorpelersatz, einen Knochenersatz oder einen Gefäßabschnitt, handeln.

Weitere Vorteile der Erfindung ergeben sich aus der Beschreibung und der Zeichnung. Die gezeigten und beschriebenen Ausführungsformen sind nicht als abschließende Aufzählung zu verstehen, sondern haben vielmehr beispielhaften Charakter für die Schilderung der Erfindung.

### Detaillierte Beschreibung der Erfindung und Zeichnung

- Fig. 1: zeigt den schematischen Aufbau eines erfindungsgemäßen Systems mit einer Vorrichtung und mit einer im Arbeitsbereich der Vorrichtung anordenbaren Polymervorstufe;
- Fig. 2: zeigt die Polymervorstufe mit der paramagnetischen Substanz in einem ausgewählten Voxel der Polymervorstufe;
- Fig. 3: zeigt eine Umhausung, innerhalb derer die Polymervorstufe während des 3D-Fertigungsprozesses angeordnet werden kann; und
- Fig. 4: zeigt ein Blockschaltbild des erfindungsgemäßen Verfahrens zum Erzeugen einer 3D-Struktur mit dessen einzelnen Verfahrensschritten.

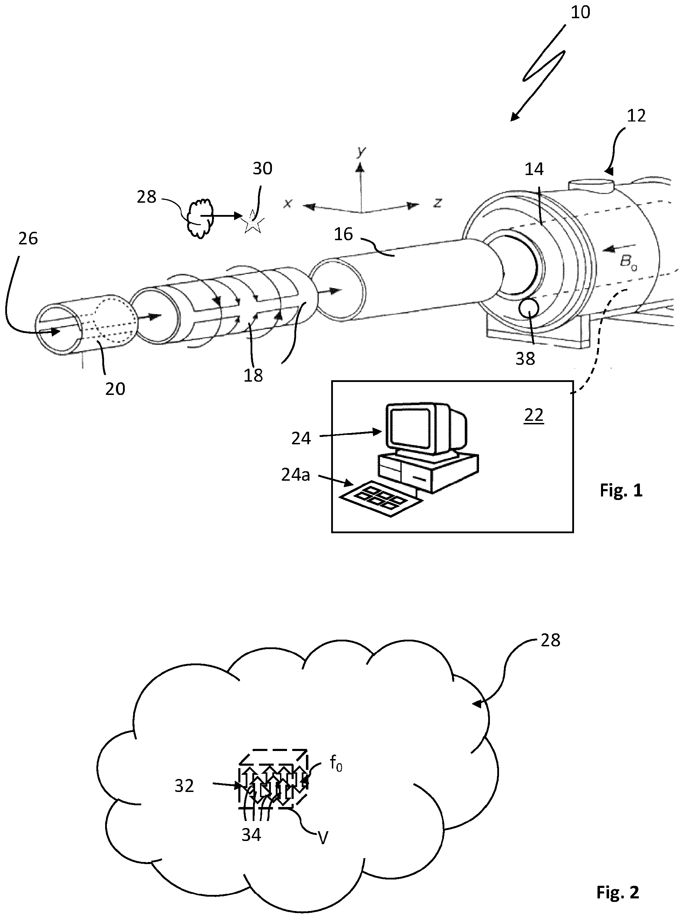

**Fig. 1** zeigt ein System **10** mit einer Vorrichtung **12,** die einen Magnetfeldgenerator **14** zum Erzeugen eines statischen Magnetfeldes B₀, optionale Shimspulen **16** und Gradientenspulen **18,** ein HF-Feldgenerator **20,** eine Steuerungseinheit **22** mit einem Computersystem **24** und einer Eingabe- und Bedienkonsole **24a,** sowie einen Arbeitsbereich **26** aufweist. In dem Arbeitsbereich 26 ist eine Polymervorstufe **28** anordenbar, die als Ausgangsmaterial für die mit der Vorrichtung 12 zu erzeugenden 3D-Struktur **30** dient.

Der Magnetfeldgenerator 14 dient dazu, im Arbeitsbereich 26 ein homogenes statisches Magnetfeld B₀ (nachfolgend kurz: B0-Feld) zu erzeugen. Die Feldstärke des B₀-Felds ist dabei um Potenzen größer, als das Erdmagnetfeld. Der Magnetfeldgenerator 14 kann beispielsweise einen Permanentmagneten oder einen supraleitenden Magneten umfassen. Dieses homogene B₀-Feld kann durch die Gradientenspulen 18 gezielt verändert werden. Die Gradientenspulen 18 befinden sich vorzugsweise am Umfang des Arbeitsbereichs 26. Mit diesen Gradientenspulen 26 können dem statischen B₀-Feld kontinuierlich ansteigende oder abfallende Magnetfelder, sogenannte Gradientenfelder, in allen drei Raumrichtungen x, y, z, überlagert werden.

Die Polymervorstufe 28 umfasst gemäß der schematischen Darstellung eines Voxels **V** der Polymervorstufe in **Fig. 2** eine paramagnetische Substanz **32,** die in der Polymervorstufe 28 vorzugsweise homogen verteilt angeordnet ist. Die paramagnetische Substanz 32 ist vorzugsweise durch superparamagnetische nanopartikuläre Metallpartikel **34** (=Nano-Oszillatoren), gebildet, die in der Polymervorstufe 28 suspendiert sind. Die Metallpartikel 34 können insbesondere aus nanopartikulärem Magnetit (Fe₃O₄) oder einem Silberhalogenid (AgₙXₙ) bestehen. Ein Milliliter der Polymervorstufe 28 enthält vorzugsweise mehr als 1000, bevorzugt mehr als 10.000, und bis zu 10¹⁷ der Metallpartikel 34.

Im statischen B₀-Feld richten alle Metallpartikel 34 ihre Rotationsmomente parallel bzw. antiparallel zu den Magnetfeldlinien des B₀-Felds aus. Damit geht eine Synchronisierung ihrer Resonanzfrequenz-Empfindlichkeit für eingestrahlte HF-Strahlung bzw. HF-Impulse einher. Die Resonanzfrequenz-Empfindlichkeit hängt neben dem Material, der Polarität und der Geometrie und Größe der Metallpartikel 34 von der Feldstärke des B₀-Felds ab. Für jede Feldstärke des B₀-Felds existiert mithin im Wesentlichen nur eine einzige, spezifische Frequenz der elektromagnetischen HF-Strahlung, welche alle in gleicher Weise konfektionierten Metallpartikel als Oszillatoren maximal stimuliert - die sogenannte Resonanzfrequenz.

Bei einfachen (theoretischen) Systemen ohne Dämpfung ist die Resonanzfrequenz gleich der ungedämpften Eigenfrequenz (Kennfrequenz) f₀ der paramagnetischen Metallpartikel 34. Bei gedämpften Systemen ist die Frequenz, bei der die maximale Amplitude auftritt, stets kleiner als die ungedämpfte Eigenfrequenz.

Unter Resonanzbedingungen weisen also alle durch die HF-Strahlung anregbaren Metallpartikel 34 eine maximale Energieabsorptionsbereitschaft hinsichtlich des spezifischen HF-Wechselfeldes auf. Dies resultiert in einer signifikant erhöhten Oszillationsbewegung der Metallpartikel 34. Diese Oszillationsbewegungen werden (auf molekularer Ebene) aufgrund von Widerstands- und Reibungseffekten mit minimalem räumlichem Kopplungsabstand in thermische Energie umgewandelt. Die Metallpartikel können also insoweit als Nano-Oszillatoren bezeichnet werden.

Die dynamischen Gradientenfelder (B1, B2, B3; in der Zeichnung nicht dargestellt) der Vorrichtung 12 dienen in einer zur Kernspintomographie entsprechenden Weise zur Schichtselektion und Ortscodierung in mindestens 3 Raumrichtungen, d.h. funktionell zur "indirekten Fokussierung" der mittels des HF-Feldgenerators 20 in den Arbeitsbereich eingestrahlten HF-Strahlung. Die Gradientenfelder sind durch einen kontinuierlichen Anstieg bzw. Abfall der jeweiligen Magnetfeldstärke entlang ihrer charakteristischen Achsen x, y, z, relativ zum B0-Feld gekennzeichnet. An dieser Stelle sei auf die dem Fachmann geläufigen und gleichartigen Gradientenfelder bei der Kernspintomografie verwiesen. Ein zugeschaltetes Gradientenfeld in der x-Achse überlagert mithin das zuvor homogene permanente statische B₀-Feld und führt somit zu einem linearen Anstieg bzw. Abfall der statischen Gesamtfeldstärke entlang der x-Achse. Gleiches gilt für jeweils zugeschaltete Gradientenfelder entlang der y-Achse und z-Achse. Dies hat zur Folge, dass jedes Metallpartikel oder jedes Raumvolumen bzw. Voxel der Polymervorstufe 28 im dreidimensionalen Raum über seine individuelle, ihm eigene elektromagnetische Nische im Schnittpunkt der mindestens 3 Gradientenfelder verfügt.

Unterscheidet sich also das magnetische Mikromilieu jedes Oszillators bzw. jedes Voxels in allen drei Raumrichtungen linear vom magnetischen Mikromilieu seines Nachbar-Oszillators bzw. benachbarten Voxels (= Nachbarvoxel), dann verfügt jedes Nanopartikel bzw. verfügen die Nanopartikel jedes einzelnen Voxels über seine/ihre ganz eigene individuelle Resonanzfrequenz und lässt/lassen sich folglich individuell isoliert adressieren und durch ein ungerichtetes elektromagnetisches HF-Feld selektiv anregen. Hierbei definiert die jeweilige Steilheit der x-, y-, z-Gradientenfelder die Kantenlängen der Voxel in allen 3 Raumrichtungen x, y, z, optimalerweise durch simultane summarische Überlagerung ihrer lokalen Feldstärke, alternativ sequentiell, z.B. unter Verwendung der inversen Fourier-Transformation.

Die Steuerungseinheit 22 der Vorrichtung 12 ist eingerichtet, den HF-Feldgenerator 20 derart anzusteuern, dass HF-Strahlung mit einer auf die Resonanzfrequenz der Metallpartikel/Oszillatoren abgestimmten Feldfrequenz in den Arbeitsbereich 26 eingestrahlt wird, um die Polymervorstufe 28 im zuvor ortscodierten Voxel V oder in der zuvor ortscodierten zusammenhängenden Volumeneinheit der Polymervorstufe "en bloc" lokal zu erhitzen und zu polymerisieren.

Die typische Feldfrequenz der HF-Strahlung zur Oszillationsanregung der paramagnetischen Substanz beträgt dabei zwischen 100 oder 130 KHz und 789 THz.

Die Steuerungseinheit 22 der Vorrichtung 12 weist vorzugsweise einen Betriebsmodus auf, der dem Gewinnen von Bilddaten (z.B. magnetresonanztomografischen Bilddaten) aus dem Arbeitsbereich 26 bzw. der Polymervorstufe 28 bzw. der bereits polymerisierten 3D-Struktur 30 dient. Die Steuerungseinheit 22, insbesondere das Computersystem 24, dient dem Steuern aller Betriebsprozesse der Vorrichtung 12 anhand von vorgegebenen CAD/CAM-Daten. Darüber hinaus ist die Steuerungseinheit 22 vorzugsweise dazu eingerichtet, die Bilddaten auszuwerten. So kann die Steuerungseinheit 22 insbesondere dazu eingerichtet sein, die CAD/CAM-Daten mit den zuvor gewonnenen Bilddaten zu vergleichen und bei Feststellen einer Abweichung, die größer ist, als eine vorgegebene zulässige maximale Abweichung, den weiteren Fertigungsprozess auf Basis der Bilddaten fortzusetzen. Hier kann die Steuerungseinheit insbesondere dazu eingerichtet (programmiert) sein, die CAD/CAM-Daten betreffend die übrige zu erzeugende 3D-Struktur zu ändern. Auf diese Weise kann die 3D-Struktur mit besonders kleinen Toleranzen gefertigt werden.

Beim in-vivo-Erzeugen (Fertigen) der 3D-Struktur 30 können darüber hinaus in Echtzeit Informationen zum an die 3D-Struktur angrenzende bzw. mit der 3D-Struktur zusammenwirkende anatomische Strukturen beim Fertigungsprozess berücksichtigt werden. Hier kann der Einsatz künstlicher Intelligenz bzw. einer Softwareapplikation mit KI-Fähigkeit von Vorteil sein, zumal im Fertigungsprozess erkannte systematische Abweichungen ggf. in Abhängigkeit von der eingesetzten Polymervorstufe, der paramagnetischen Substanz, den Umgebungsvariablen etc. berücksichtigt und bei der Erstellung/Änderung der CAD/CAM Daten für die betreffende 3D-Struktur und/oder den Fertigungsprozess prospektiv berücksichtigt werden können.

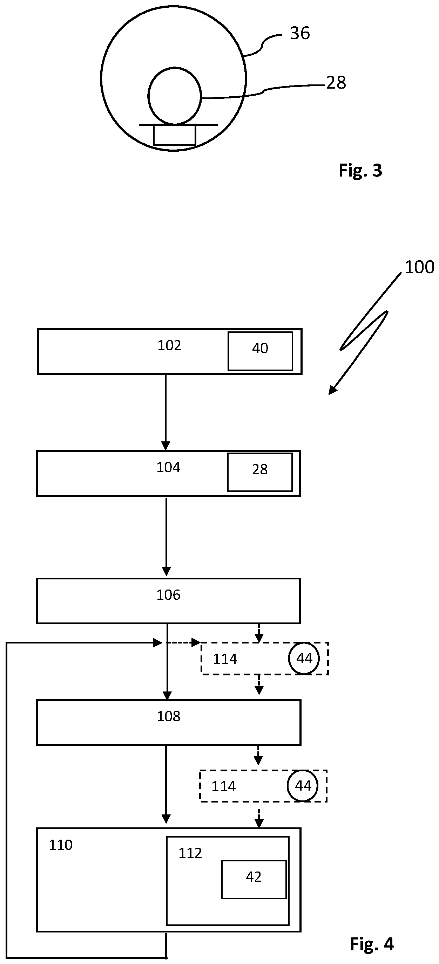

Der Arbeitsbereich 26 der Vorrichtung kann gemäß Fig. 3 mittels einer, bevorzugt gasdichten, Umhausung **36** abgegrenzt sein. Die Umhausung 36 kann beispielsweise aus Kunststoff oder aus Glas oder einem anderen gegenüber HF-Feldern bzw. Magnetfeldern nicht-schirmenden Material gebildet sein. Die Umhausung kann beispielsweise durch eine Kunststofffolie gebildet sein, in der die Polymervorstufe 28 angeordnet wird/ist.

Dem Arbeitsbereich der Vorrichtung 12 kann eine Pumpe **38** (Fig. 1) zugeordnet sein, mittels derer die Atmosphäre in der Umhausung 36 evakuierbar oder im Wesentlichen evakuierbar ist und/oder über die der Arbeitsbereich innerhalb der Umhausung 36 mit einem für den Fertigungsprozess vorgegebenen Fluid, insbesondere einer Arbeitsatmosphäre A, befüllbar ist. Auf diese Weise kann beispielsweise unerwünschten oxidativen Prozessen der Polymervorstufe 28 durch in der Arbeitsatmosphäre A enthaltenen Sauerstoff entgegengewirkt werden.

Die Vorrichtung 12 kann insbesondere durch ein modifiziertes MRT-Gerät gebildet sein, dessen Steuerungseinheit 22 in der vorstehend erläuterten Weise an die Fertigung des 3D-Struktur adaptiert ist.

Die Polymervorstufe 28 kann gleiche oder unterschiedliche Monomere bzw. Polymere (insbesondere auch Dimere, Oligomere) aufweisen. Darüber hinaus kann die Polymervorstufe 28 Fasern und/oder einen oder mehrere andere Zuschlagstoffe, wie diese eingangs erläutert sind, umfassen. Rein beispielhaft seien hier Zuschlagstoffe aus der Gruppe der Farbstoffe, der antibakteriellen Substanzen, der Antibiotika oder der Wachstumsfaktoren, umfassen.

In Abhängigkeit von den an die 3D-Struktur gestellten mechanischen, elektrischen oder biologischen Anforderungen kann die Polymervorstufe 28 eine Viskosität von ungefähr 10² mPa·s bis 10⁵ mPa·s oder größer aufweisen. Soll die 3D-Struktur 30 beispielsweise als Implantat am Menschen/Tier eingesetzt werden, so ist die Polymervorstufe 28 im nicht-auspolymerisierten Zustand vorzugsweise für den tierischen/menschlichen Organismus nicht toxisch und bevorzugt durch körpereigene Enzyme abbaubar bzw. per viam naturalis aus dem menschlichen/tierischen Körper eliminierbar.

Die Vorrichtung 12 kann universell eingesetzt werden. So können damit beispielsweise medizinische Implantate, insbesondere Knochenersatz, Traggerüste für Gewebe/Organe oder Gefäßprothesen erzeugt werden. Dies kann in vitro oder auch direkt in vivo erfolgen.

Nachstehend ist das Verfahren 100 zum Erzeugen einer 3D-Struktur unter zusätzlicher Bezugnahme auf das in Fig. 4 gezeigte Blockschaubild näher erläutert.

Das Verfahren 100 zum Erzeugen der 3D-Struktur 30 (Fig. 1) setzt zwingend den Einsatz des vorstehend im Kontext mit den Fign. 1 bis 4 erläuterten Systems 10 mit der Vorrichtung 12 und mit der erfindungsgemäßen Polymervorstufe 28 voraus und umfasst die folgenden Schritte:
a. Definieren **102** von CAD/CAM-Daten **40** zu der zu fertigenden 3D-Struktur 30;
b. Bereitstellen **104** einer Polymervorstufe 28 mit einer darin möglichst homogen verteilten paramagnetischen Substanz 32;
c. Einbringen **106** der Polymervorstufe 28 in den Arbeitsbereich der Vorrichtung;
d. Ortskodieren **108** eines Voxels V innerhalb der Polymervorstufe 28 in Abhängigkeit von den CAD/CAM-Daten durch Anlegen magnetischer Gradientenfelder;
e. Polymerisieren **110** der Polymervorstufe 28 in dem zumindest einen ortskodierten Voxel V durch Einstrahlen **112** von HF-Strahlung **42**, mittels derer die paramagnetische Substanz 32 in dem jeweiligen weiteren Voxel V zu Oszillationen angeregt wird; und
f. Nachfolgend sequentielles Ortskodieren 108 weiterer, bevorzugt räumlich jeweils aneinander angrenzender, Voxel V in der Polymervorstufe 28 in Abhängigkeit von den CAD/CAM-Daten 40 und Polymerisieren 110 der jeweilig weiteren ortscodierten Voxel V durch Einstrahlen **112** von HF-Strahlung 42, mittels derer die paramagnetische Substanz 32 in dem jeweiligen weiteren Voxel V zu Oszillationen angeregt wird.

Die Frequenz der HF-Strahlung **42**, d. h. des angelegten HF-Felds, ist vorzugsweise auf die jeweilige Resonanzfrequenz f₀ der mit der HF-Strahlung anzuregenden paramagnetischen Substanz 32 bzw. die Metallpartikel 34 der Polymervorstufe 28 abgestimmt. Die Resonanzfrequenz f₀ der jeweiligen paramagnetischen Substanz 32 kann experimentell bestimmt werden.

Zu beachten ist, dass die Voxel V jeweils eine einheitliche Größe aufweisen können oder sich in ihrer Größe zumindest teilweise voneinander unterscheiden können.

Nach einer Weiterbildung der Erfindung werden betreffs der Polymervorstufe 28 in einem optionalen Schritt **114,** insbesondere magnetresonanztomografisch oder im Wege eines alternativen Bildgebungsverfahrens, Bilddaten 44 der nichtpolymerisierten und/oder der polymerisierten Polymervorstufe 28, gewonnen. Dieser Schritt kann insbesondere, vor (insbesondere während Schritt c)) und/oder nach dem vorstehend angegebenen Schritt d), insbesondere nach dem Schritt e) sowie vorzugsweise in Abhängigkeit von den CAD/CAM Daten 40 erfolgen.

Die Bilddaten 44 können dabei in einem optionalen Schritt mit den CAD/CAM Daten 40 verglichen und die CAD/CAM-Daten 40 für die 3D-Struktur 30 bei Überschreiten einer maximalen Abweichung des bereits erzeugten 3D-Struktur 30 auf Grundlage der Bilddaten für das Erzeugen der übrigen 3D-Struktur 30 geändert werden. Auf diese Weise kann die 3D-Struktur 30 mit einer besonders geringen Toleranz erzeugt werden.

Für medizinische Indikationen kann die 3D-Struktur 30 vollständig in einem ersten Lebewesen (in der Zeichnung nicht gezeigt), d.h. in-vivo, gedruckt werden, um nach dessen Entnahme als Implantat für ein anderes Lebewesen (nicht gezeigt) zur Verfügung zu stehen.

Mittels des erfindungsgemäßen Verfahrens 100 können wie eingangs beschrieben, beliebige 3D-Strukturen, beispielsweise Maschinenteile, Traggerüste für Zellen, Gewebe, Organe usw. erzeugt werden.

## Patentansprüche

1. Vorrichtung (12) zum Erzeugen einer 3D-Struktur (30),
umfassend:
• einen Magnetfeldgenerator (14) zum Erzeugen eines statischen Magnetfeldes B₀ in einem Arbeitsbereich (26) der Vorrichtung (12), in dem eine Polymervorstufe (28) umfassend zumindest eine paramagnetische Substanz (32) anordenbar ist;
• Gradientenspulen (18) zum Erzeugen von magnetischen Gradientenfeldern in allen drei Raumrichtungen x, y, z, mittels derer die paramagnetische Substanz (32) in einem definierten Voxel V der Polymervorstufe (28) ortscodierbar ist;
• einen Hochfrequenz- (HF-) Feldgenerator (20) zum Einstrahlen von HF-Strahlung (42) in den Arbeitsbereich (26); und
• eine Steuerungseinheit (22), die dazu eingerichtet ist, den HF-Feldgenerator (20) derart anzusteuern, dass die ortskodierte paramagnetische Substanz (32) im dem Voxel V mittels einer auf die paramagnetische Substanz (32) abgestimmten Feldfrequenz der HF-Strahlung (42) anregbar ist, um die Polymerisation der Polymervorstufe (28) in dem definierten Voxel V auszulösen.

2. Vorrichtung (12) gemäß Anspruch 1, **dadurch gekennzeichnet, dass** die Feldfrequenz zwischen 100 KHz oder 130 KHz und 789 THz beträgt.

3. Vorrichtung (12) gemäß Anspruch 1 oder 2, **dadurch gekennzeichnet, dass** die Steuerungseinheit (22) einen Betriebsmodus zum Gewinnen von, insbesondere magnetresonanztomografischen, Bilddaten (44), aus dem Arbeitsbereich (26) aufweist.

4. Vorrichtung (12) gemäß Anspruch 3, **dadurch gekennzeichnet, dass** die Steuerungseinrichtung (22) dazu eingerichtet ist, die Bilddaten (44) mit den CAD/CAM Daten (40) für die 3D-Struktur (30) zu vergleichen und bei Feststellen von, insbesondere geometrischen, Abweichungen des teilerzeugten 3D-Struktur (30) von den CAD/CAM-Daten (40) die Bilddaten (44) bzw. die Abweichungen beim weiteren Drucken der 3D-Struktur (30) zu berücksichtigen.

5. Vorrichtung (12) gemäß Anspruch 4, **dadurch gekennzeichnet, dass** die Steuerungseinheit (22) dazu eingerichtet ist, die CAD/CAM-Daten (40) auf Grundlage der Bilddaten (44) zu ändern.

6. Vorrichtung (12) gemäß einem der vorhergehenden Ansprüche, **dadurch gekennzeichnet, dass** der Arbeitsbereich (26) innerhalb einer Umhausung (36) angeordnet ist.

7. Vorrichtung (12) gemäß Anspruch 6, **dadurch gekennzeichnet, dass** die Umhausung (36) eine Kunststofffolie oder Glas umfasst.

8. Vorrichtung (12) gemäß einem der vorhergehenden Ansprüche, **dadurch gekennzeichnet, dass** die Vorrichtung (12) eine Pumpe (38) aufweist, mittels derer die Umgebungsatmosphäre im Arbeitsbereich (26) evakuierbar oder im Wesentlichen evakuierbar ist und/oder über die der Arbeitsbereich (26) mit einer für den Fertigungsprozess vorgegebenen Arbeitsatmosphäre A bzw. einer Flüssigkeit befüllbar ist.

9. Vorrichtung (12) gemäß einem der vorhergehenden Ansprüche, **dadurch gekennzeichnet, dass** die Vorrichtung eine Temperiereinrichtung zum Temperieren des Arbeitsbereichs (26) aufweist.

10. Vorrichtung (12) gemäß einem der vorhergehenden Ansprüche, **dadurch gekennzeichnet, dass** die Vorrichtung (12) ein MPT-Gerät oder eine andere Bildgebungseinheit umfasst.

11. System (10) zum Erzeugen einer 3D-Struktur, umfassend eine Vorrichtung (12) gemäß einem der vorhergehenden Ansprüche und die Polymervorstufe (28) mit zumindest einer paramagnetischen Substanz (32).

12. System (10) gemäß Anspruch 11, **dadurch gekennzeichnet, dass** die paramagnetische Substanz (32) Metallpartikel (34) oder Metallorganyle umfasst.

13. System (10) gemäß Anspruch 12, **dadurch gekennzeichnet, dass** die Metallpartikel (34) nanopartikuläre Magnetitpartikel oder nanopartikuläre Eisenpartikel umfassen.

14. System (10) gemäß Anspruch 12 oder 13, **dadurch gekennzeichnet, dass** die Metallpartikel (34) in einer Konzentration von > 1000 Partikel pro Milliliter der Polymervorstufe, insbesondere in einer Konzentration von > 10.000 Partikel pro Milliliter der Polymervorstufe, enthalten sind.

15. System (10) gemäß einem der Ansprüche 12 bis 14, **dadurch gekennzeichnet, dass** sich zumindest ein Teil der Metallpartikel (34) voneinander unterscheiden, insbesondere eine unterschiedliche Größe oder Form aufweisen.

16. System (10) gemäß einem der vorhergehenden Ansprüche 11 bis 15, **dadurch gekennzeichnet, dass** die Metallpartikel (34) jeweils, insbesondere mit Titan, beschichtet sind.

17. System (10) gemäß einem der Ansprüche 11 bis 16, **dadurch gekennzeichnet, dass** die Polymervorstufe (28) zumindest zwei unterschiedliche Monomere oder unterschiedliche Polymere umfasst.

18. System (10) gemäß einem der Ansprüche 11 bis 17, **dadurch gekennzeichnet, dass** die Polymervorstufe Polysaccharide, z. B. Alginate, und/oder Methacrylsäure und/oder Polylactide (PLA-Derivate) und/oder ECM-Derivate und/oder (bio-)artifizielle Polymere umfasst.

19. System (10) gemäß einem der Ansprüche 11 bis 18, **dadurch gekennzeichnet, dass** die Polymervorstufe (28) einen oder mehrere Zuschlagstoffe, insbesondere aus der Gruppe der Fasern, Farbstoffe, antibakteriellen Substanzen, Wachstumsfaktoren, Nanopartikel/-tubes, mineralischen Füllstoffe, metallischen Werkstoffe, Glykosaminoglykane, MMC-Stoffe, Polypeptid-Motive, Promotoren, Terminatoren, Inhibitoren, Katalysatoren, Sensitizer und/oder Immunmodulatoren umfasst.

20. System (10) gemäß einem der Ansprüche 11 bis 19, **dadurch gekennzeichnet, dass** die Polymervorstufe eine Viskosität von 10² Pa·s bis 10⁶ Pa·s aufweist.

21. System (10) gemäß einem der Ansprüche 11 bis 20, **dadurch gekennzeichnet, dass** die Polymervorstufe (28) im nicht-auspolymerisierten Zustand im menschlichen und/oder tierischen Körper, vorzugsweise durch körpereigene Enzyme, abbaubar bzw. aus dem Körper per viam naturalis ausscheidbar ist.

22. Verwendung einer Vorrichtung (12) gemäß einem der Ansprüche 1 bis 10 und/oder eines Systems (10) gemäß einem der Ansprüche 11 bis 21 zum Erzeugen einer 3D-Struktur (30) in Form eines Maschinenelements oder eines medizinischen Implantats, insbesondere eines Knochenersatzes, eines Traggerüsts für Zellen, eines Gewebes oder eines Organs oder einer Gefäßprothese, wobei das Erzeugen der 3D-Struktur nicht in-vivo erfolgt.

23. Verfahren (100) zum Erzeugen einer 3D-Struktur (30) mittels eines Systems (10) gemäß einem der Ansprüche 11 bis 21, umfassend die folgenden Schritte:
a. Definieren (102) von CAD/CAM-Daten (40) zu der zu fertigenden 3D-Struktur (30);
b. Bereitstellen (104) einer Polymervorstufe (28), die eine, bevorzugt homogen verteilte, paramagnetische Substanz umfasst;
c. Einbringen (106) der Polymervorstufe (28) in den Arbeitsbereich der Vorrichtung (12);
d. Ortskodieren (108) eines Voxels V innerhalb der Polymervorstufe (28) in Abhängigkeit von den CAD/CAM Daten (40) durch Anlegen magnetischer Gradientenfelder;
e. Polymerisieren (110) der Polymervorstufe (28) in dem zumindest einen ortskodierten Voxel V durch Einstrahlen (112) von HF-Strahlung (42) mittels derer die paramagnetische Substanz 32 in dem jeweiligen weiteren Voxel V zu Oszillationen angeregt wird; und
f. Nachfolgend sequentielles Ortskodieren (108) weiterer, bevorzugt räumlich jeweils aneinander angrenzender, Voxel V in der Polymervorstufe (28) in Abhängigkeit von den CAD/CAM-Daten (40) und Polymerisieren (110) der jeweilig weiteren ortscodierten Voxel V durch Einstrahlen (112) von HF-Strahlung (42), mittels derer die paramagnetische Substanz (32) in dem jeweiligen weiteren Voxel V zu Oszillationen angeregt wird,
wobei das Erzeugen der 3D-Struktur nicht in-vivo erfolgt.

24. Verfahren (100) gemäß Anspruch 23, **dadurch gekennzeichnet, dass** die Frequenz der HF-Strahlung (42), d. h. des angelegten HF-Feldes, in Abhängigkeit von der (bekannten) Resonanzfrequenz f₀ der mit der HF-Strahlung (42) anzuregenden paramagnetischen Substanz (32) der Polymervorstufe (28) gewählt wird.

25. Verfahren (100) gemäß Anspruch 23 oder 24, **dadurch gekennzeichnet, dass** die Voxel V jeweils mit einer einheitlichen (Volumen-)Größe definiert werden oder dass die Voxel V zumindest teilweise mit einer unterschiedlichen (Volumen-)Größe definiert werden.

26. Verfahren (100) gemäß einem der Ansprüche 23 bis 25, **dadurch gekennzeichnet, dass** zur Polymervorstufe (28) und/oder zu dem teilerzeugten 3D-Struktur (30), insbesondere magnetresonanztomografisch, (Bild-)Daten (44), gewonnen werden und der weitere Fertigungsprozess unter Berücksichtigung dieser magnetresonanztomografischen Daten erfolgt.

27. Verfahren (100) gemäß einem der Ansprüche 23 bis 26, **gekennzeichnet durch** die weiteren Schritte Vergleichen (114) der Bilddaten (44) mit den CAD/CAM Daten (40); und Ändern der CAD/CAM-Daten (40) für den 3D-Struktur bei Überschreiten einer maximalen Abweichung der Bilddaten (44) von den CAD/CAM Daten (40) auf Grundlage der Bilddaten (44).

28. Verfahren (100) gemäß einem der Ansprüche 23 bis 27, **dadurch gekennzeichnet, dass** das Verfahren (100) zum Erzeugen eines 3D-Struktur (30) in Form eines Maschinenelements oder eines Implantats verwendet wird.

## Claims

1. An apparatus (12) for generating a 3D structure (30),
comprising:
• a magnetic field generator (14) for generating a static magnetic field B₀ in a working zone (26) of the apparatus (12), in which a polymer precursor (28) comprising at least one paramagnetic substance (32) can be arranged;
• gradient coils (18) for generating magnetic gradient fields in all three spatial directions x, y, z, by means of which gradient coils the paramagnetic substance (32) can be spatially encoded in a defined voxel V of the polymer precursor (28);
• a radio-frequency (RF) field generator (20) for irradiating RF radiation (42) into the working zone (26); and
• a control unit (22) which is configured to control the RF field generator (20) in such a way that the spatially encoded paramagnetic substance (32) in the voxel V can be excited by means of a field frequency of the RF radiation (42) tuned to the paramagnetic substance (32) in order to trigger the polymerization of the polymer precursor (28) in the defined voxel V.

2. The apparatus (12) according to claim 1, **characterized in that** the field frequency is between 100 KHz or 130 KHz and 789 THz.

3. The apparatus (12) according to either claim 1 or claim 2, **characterized in that** the control unit (22) has an operating mode for obtaining image data (44) from the working zone (26), in particular magnetic resonance tomography image data.

4. The apparatus (12) according to claim 3, **characterized in that** the control device (22) is configured to compare the image data (44) with the CAD/CAM data (40) for the 3D structure (30) and, if deviations of the partially-generated 3D structure (30) from the CAD/CAM data (40) are detected, in particular geometric deviations, to take into account the image data (44) and/or the deviations during the further printing of the 3D structure (30).

5. The apparatus (12) according to claim 4, **characterized in that** the control unit (22) is configured to change the CAD/CAM data (40) on the basis of the image data (44).

6. The apparatus (12) according to any of the preceding claims, **characterized in that** the working zone (26) is arranged within a housing (36).

7. The apparatus (12) according to claim 6, **characterized in that** the housing (36) comprises a plastic film or glass.

8. The apparatus (12) according to any of the preceding claims, **characterized in that** the apparatus (12) has a pump (38) by means of which the ambient atmosphere in the working zone (26) can be evacuated or substantially evacuated and/or via which the working zone (26) can be filled with a working atmosphere A and/or a fluid that is prespecified for the production process.

9. The apparatus (12) according to any of the preceding claims, **characterized in that** the apparatus has a temperature control device for controlling the temperature of the working zone (26).

10. The apparatus (12) according to any of the preceding claims, **characterized in that** the apparatus (12) comprises an MRT device or a different imaging unit.

11. A system (10) for generating a 3D structure, comprising an apparatus (12) according to any of the preceding claims and a polymer precursor (28) with at least one paramagnetic substance (32).

12. The system (10) according to claim 11, **characterized in that** the paramagnetic substance (32) comprises metal particles (34) or metal organyls.

13. The system (10) according to claim 12, **characterized in that** the metal particles (34) comprise nanoparticulate magnetite particles or nanoparticulate iron particles.

14. The system (10) according to claim 12 or 13, **characterized in that** the metal particles (34) are present in a concentration of > 1000 particles per milliliter of the polymer precursor, in particular in a concentration of > 10,000 particles per milliliter of the polymer precursor.

15. The system (10) according to any of claims 12 to 14, **characterized in that** at least a portion of the metal particles (34) differ from one another, in particular by having a different size or shape.

16. The system (10) according to any of the preceding claims 11 to 15, **characterized in that** the metal particles (34) are each coated, in particular with titanium.

17. The system (10) according to any of claims 11 to 16, **characterized in that** the polymer precursor (28) comprises at least two different monomers or different polymers.

18. The system (10) according to any of claims 11 to 17, **characterized in that** the polymer precursor comprises polysaccharides, e.g. alginates, and/or methacrylic acid and/or polylactides (PLA derivatives) and/or ECM derivatives and/or (bio)artificial polymers.

19. The system (10) according to any of claims 11 to 18, **characterized in that** the polymer precursor (28) comprises one or more additives, in particular from the group of fibers, dyes, antibacterial substances, growth factors, nanoparticles/tubes, mineral fillers, metallic materials, glycosaminoglycans, MMC substances, polypeptide motifs, promoters, terminators, inhibitors, catalysts, sensitizers, and/or immunomodulators.

20. The system (10) according to any of claims 11 to 19, **characterized in that** the polymer precursor has a viscosity of 10² mPa·s to 10⁶ mPa·s.

21. The system (10) according to any of claims 11 to 20, **characterized in that** the polymer precursor (28) can be broken down and/or removed from the body by natural pathways in the non-polymerized state in the human and/or animal body, preferably by endogenous enzymes.

22. A use of an apparatus (12) according to any of claims 1 to 10 and/or a system (10) according to any of claims 11 to 21 for producing a 3D structure (30) in the form of a machine element or a medical implant, in particular a bone replacement, a supporting framework for cells, a tissue, or an organ or a vascular prosthesis, whereat the production of the 3D structure is not carried out in-vivo.

23. A method (100) for producing a 3D structure (30) by means of a system (10) according to any of claims 11 to 21, comprising the following steps:
a. defining (102) CAD/CAM data (40) for the 3D structure to be produced (30);
b. providing (104) a polymer precursor (28) comprising a preferably homogeneously distributed paramagnetic substance;
c. introducing (106) the polymer precursor (28) into the working zone of the apparatus (12);
d. spatially encoding (108) a voxel V within the polymer precursor (28) as a function of the CAD/CAM data (40) by applying magnetic gradient fields;
e. polymerizing (110) the polymer precursor (28) in the at least one spatially-encoded voxel V by irradiating (112) RF radiation (42) by means of which the paramagnetic substance (32) is excited to oscillations in the corresponding further voxel V; and
f. subsequently sequentially spatially encoding (108) further voxels V, preferably spatially adjacent to one another, in the polymer precursor (28) as a function of the CAD/CAM data (40), and polymerizing (110) the corresponding further spatially-encoded voxels V by irradiation (112) of RF radiation (42) by means of which the paramagnetic substance (32) is excited to oscillations in the corresponding further voxel V, whereat the production of the 3D structure is not carried out in-vivo.

24. The method (100) according to claim 23, **characterized in that** the frequency of the RF radiation (42), i.e., of the applied RF field, is selected depending on the (known) resonant frequency f₀ of the paramagnetic substance (32) of the polymer precursor (28) to be excited with the RF radiation (42).

25. The method (100) according to claim 23 or 24, **characterized in that** the voxels V are each defined with a uniform (volume) size, or **in that** the voxels V are at least partially defined with a different (volume) size.

26. The method (100) according to any of claims 23 to 25, **characterized in that** (image) data (44) are obtained of the polymer precursor (28) and/or the partially generated 3D structure (30), in particular magnetic resonance tomography data, and the further manufacturing process takes place taking into account this magnetic resonance tomography data.

27. The method (100) according to any of claims 23 to 26, **characterized by** the further steps of comparing (114) the image data (44) with the CAD/CAM data (40), and changing the CAD/CAM data (40) for the 3D structure if a maximum deviation of the image data (44) from the CAD/CAM data (40) is exceeded, on the basis of the image data (44).

28. The method (100) according to any of claims 23 to 27, **characterized in that** the method (100) is used to generate a 3D structure (30) in the form of a machine element or an implant.

## Revendications

1. Dispositif (12) pour la génération d'une structure 3D (30), comprenant :
• un générateur de champ magnétique (14) pour la génération d'un champ magnétique statique Bo dans une zone de travail (26) du dispositif (12), dans laquelle un précurseur de polymère (28) comprenant au moins une substance paramagnétique (32) peut être disposé ;
• des bobines de gradient (18) pour la génération de champs de gradient magnétiques dans toutes les trois directions spatiales x, y, z, au moyen desquels la substance paramagnétique (32) peut être codée localement dans un voxel V défini du précurseur de polymère (28) ;
• un générateur de champ haute fréquence (HF) (20) pour l'irradiation d'un rayonnement HF (42) dans la zone de travail (26) ; et
• une unité de commande (22), qui est conçue pour commander le générateur de champ HF (20) de telle sorte que la substance paramagnétique (32) codée localement dans le voxel V puisse être excitée au moyen d'une fréquence de champ du rayonnement HF (42) adaptée à la substance paramagnétique (32), afin de déclencher la polymérisation du précurseur de polymère (28) dans le voxel V défini.

2. Dispositif (12) selon la revendication 1, **caractérisé en ce que** la fréquence de champ se situe entre 100 KHz ou 130 KHz et 789 THz.

3. Dispositif (12) selon la revendication 1 ou 2, **caractérisé en ce que** l'unité de commande (22) présente un mode de fonctionnement pour l'acquisition de données d'image (44), en particulier de tomographie par résonance magnétique, à partir de la zone de travail (26).

4. Dispositif (12) selon la revendication 3, **caractérisé en ce que** le dispositif de commande (22) est conçu pour comparer les données d'image (44) avec les données CAD/CAM (40) pour la structure 3D (30) et en cas de constatations d'écarts, en particulier géométriques, de la structure 3D (30) partiellement produite par rapport aux données CAD/CAM (40), pour prendre en compte les données d'image (44) ou les écarts lors de la poursuite de l'impression de la structure 3D (30).

5. Dispositif (12) selon la revendication 4, **caractérisé en ce que** l'unité de commande (22) est conçue pour modifier les données CAD/CAM (40) sur la base des données d'image (44).

6. Dispositif (12) selon l'une des revendications précédentes,
**caractérisé en ce que** la zone de travail (26) est disposée à l'intérieur d'une enceinte (36).

7. Dispositif (12) selon la revendication 6, **caractérisé en ce que** l'enceinte (36) comprend un film plastique ou du verre.

8. Dispositif (12) selon l'une des revendications précédentes,
**caractérisé en ce que** le dispositif (12) présente une pompe (38), au moyen de laquelle l'atmosphère ambiante dans la zone de travail (26) peut être évacuée ou sensiblement évacuée et/ou par l'intermédiaire de laquelle la zone de travail (26) peut être remplie d'un liquide ou d'une atmosphère de travail A prédéfinie pour le processus de fabrication.

9. Dispositif (12) selon l'une des revendications précédentes,
**caractérisé en ce que** le dispositif présente un dispositif de régulation de température pour la régulation de la température de la zone de travail (26).

10. Dispositif (12) selon l'une des revendications précédentes,
**caractérisé en ce que** le dispositif (12) comprend un appareil d'IRM ou une autre unité d'imagerie.

11. Système (10) pour la génération d'une structure 3D, comprenant un dispositif (12) selon l'une des revendications précédentes et le précurseur de polymère (28) comportant au moins une substance paramagnétique (32).

12. Système (10) selon la revendication 11, **caractérisé en ce que** la substance paramagnétique (32) comprend des particules métalliques (34) ou des organyles métalliques.

13. Système (10) selon la revendication 12, **caractérisé en ce que** les particules métalliques (34) comprennent des particules de magnétite nanoparticulaires ou des particules de fer nanoparticulaires.

14. Système (10) selon la revendication 12 ou 13, **caractérisé en ce que** les particules métalliques (34) sont contenues en une concentration de > 1000 particules par millilitre du précurseur de polymère, en particulier en une concentration de > 10 000 particules par millilitre du précurseur de polymère.

15. Système (10) selon l'une des revendications 12 à 14,
**caractérisé en ce que** au moins une partie des particules métalliques (34) se différencient les unes des autres, en particulier présentent une taille ou une forme différente.

16. Système (10) selon l'une des revendications précédentes 11 à 15,
**caractérisé en ce que** les particules métalliques (34) sont respectivement revêtues, en particulier avec du titane.

17. Système (10) selon l'une des revendications 11 à 16,
**caractérisé en ce que** le précurseur de polymère (28) comprend au moins deux monomères différents ou polymères différents.

18. Système (10) selon l'une des revendications 11 à 17,
**caractérisé en ce que** le précurseur de polymère comprend des polysaccharides, par ex. des alginates, et/ou de l'acide méthacrylique et/ou des polylactides (dérivés de PLA) et/ou des dérivés d'ECM et/ou des polymères (bio)artificiels.

19. Système (10) selon l'une des revendications 11 à 18,
**caractérisé en ce que** le précurseur de polymère (28) comprend un ou plusieurs additifs, en particulier du groupe des fibres, colorants, substances antibactériennes, facteurs de croissance, nanoparticules/tubes, charges minérales, matériaux métalliques, glycosaminoglycanes, substances MMC, motifs polypeptidiques, promoteurs, terminateurs, inhibiteurs, catalyseurs, sensibilisateurs et/ou immunomodulateurs.

20. Système (10) selon l'une des revendications 11 à 19,
**caractérisé en ce que** le précurseur de polymère présente une viscosité de 102 Pa·s à 106 Pa·s.

21. Système (10) selon l'une des revendications 11 à 20,
**caractérisé en ce que** le précurseur de polymère (28) à l'état non polymérisé est dégradable dans le corps humain et/ou animal, de préférence par des enzymes propres au corps ou peut être éliminé du corps par des voies naturelles.

22. Utilisation d'un dispositif (12) selon l'une des revendications 1 à 10 et/ou d'un système (10) selon l'une des revendications 11 à 21 pour la génération d'une structure 3D (30) sous la forme d'un élément de machine ou d'un implant médical, en particulier d'un substitut osseux, d'une structure de support pour cellules, d'un tissu ou d'un organe ou d'une prothèse vasculaire, dans laquelle la génération de la structure 3D n'est pas effectuée in vivo.

23. Procédé (100) pour la génération d'une structure 3D (30) au moyen d'un système (10) selon l'une des revendications 11 à 21, comprenant les étapes suivantes :
a. définition (102) de données CAD/CAM (40) pour la structure 3D (30) à fabriquer ;
b. fourniture (104) d'un précurseur de polymère (28), qui comprend une substance paramagnétique, de préférence répartie de manière homogène ;
c. introduction (106) du précurseur de polymère (28) dans la zone de travail du dispositif (12) ;
d. codage local (108) d'un voxel V à l'intérieur du précurseur de polymère (28) en fonction des données CAD/CAM (40) par application de champs de gradient magnétiques ;
e. polymérisation (110) du précurseur de polymère (28) en l'autre moins un voxel V codé localement par irradiation (112) de rayonnement HF (42) au moyen duquel la substance paramagnétique 32 est excitée en oscillations dans l'autre voxel V respectif ; et
f. ensuite codage local séquentiel (108) d'autres voxels V, de préférence respectivement contigus dans l'espace les uns par rapport aux autres dans le précurseur de polymère (28) en fonction des données CAD/CAM (40) et polymérisation (110) des autres voxels V localement respectifs par irradiation (112) de rayonnement HF (42), au moyen duquel la substance paramagnétique (32) est excitée en oscillations dans l'autre voxel V respectif,
dans lequel la génération de la structure 3D n'est pas effectuée in vivo.

24. Procédé (100) selon la revendication 23, **caractérisé en ce que** la fréquence du rayonnement HF (42), c'est-à-dire du champ HF appliqué, est choisie en fonction de la fréquence de résonance f₀ (connue) de la substance paramagnétique (32) du précurseur de polymère (28) à exciter avec le rayonnement HF (42).

25. Procédé (100) selon la revendication 23 ou 24, **caractérisé en ce que** les voxels V sont respectivement définis avec une taille (volumique) uniforme ou que les voxels V sont définis au moins partiellement avec une taille (volumique) différente.

26. Procédé (100) selon l'une des revendications 23 à 25,
**caractérisé en ce que** des données (d'image) (44), en particulier par tomographie par résonance magnétique, relatives au précurseur de polymère (28) et/ou à la structure 3D (30) partiellement générée, sont obtenues et la suite du processus de fabrication s'effectue en tenant compte de ces données de tomographie par résonance magnétique.

27. Procédé (100) selon l'une des revendications 23 à 26, **caractérisé par** l'étape supplémentaire de comparaison (114) des données d'image (44) aux données CAD/CAM (40) ; et de modification des données CAD/CAM (40) pour la structure 3D en cas de dépassement d'un écart maximal des données d'image (44) par rapport aux données CAD/CAM (40) sur la base des données d'image (44).

28. Procédé (100) selon l'une des revendications 23 à 27,
**caractérisé en ce que** le procédé (100) est utilisé pour la génération d'une structure 3D (30) sous la forme d'un élément de machine ou d'un implant.
